# EUROPÄISCHE PATENTSCHRIFT

(11) **EP 3 831 426 B1**
(45) Veröffentlichungstag und Bekanntmachung des Hinweises auf die Patenterteilung: **09.07.2025**
(21) Anmeldenummer: 19213716.4
(22) Anmeldetag: 05.12.2019
(51) Int. Cl.: A61M 5/46

(54) **VORRICHTUNG ZUR LOKALEN APPLIKATION VON PHARMAZEUTISCHEN FLUIDEN**
DEVICE FOR LOCAL APPLICATION OF PHARMACEUTICAL FLUIDS
DISPOSITIF D'APPLICATION LOCALE DE FLUIDES PHARMACEUTIQUES

(43) Veröffentlichungstag der Anmeldung: 09.06.2021
(73) Patentinhaber: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Schultheiss & Sterzel Patentanwälte PartG mbB

(56) Entgegenhaltungen:
- WO-A1-2014/049887
- US-A- 4 861 341
- US-A1- 2003 158 520
- US-A1- 2019 275 237

## Beschreibung

Die Erfindung betrifft eine Vorrichtung zur lokalen Applikation von pharmazeutischen Fluiden sowie ein Verfahren zur Vorbereitung einer solchen Vorrichtung vor einer medizinischen Anwendung.

Gegenstand der Erfindung ist insbesondere eine medizinische Vorrichtung zur temporären, lokalen Applikation von pharmazeutischen Fluiden beziehungsweise von medizinischen Fluiden über einen Zeitraum von einigen Stunden bis zu mehreren Tagen. Die erfindungsgemäße Vorrichtung ist vor allem für die Prävention und auch zur Behandlung von periprothetischen Infektionen von Frakturen bestimmt. Die erfindungsgemäße Vorrichtung ist als Applikationssystem für eine lokale Applikationen von pharmazeutischen Fluiden im und am Knochengewebe, insbesondere im Markraum, über einen Zeitraum von mehreren Stunden bis Tagen vorgesehen und geeignet.

Aus der WO 2014/049887 A1 ist eine Spritze für Injektionen bekannt, bei der eine äußere Nadel gegen eine innere Nadel bewegt wird, wobei nach einem Einstich nur die innere Nadel im Patienten verbleibt, während die äußere Nadel zurückgezogen wird. Die US 2019/027237 A1 offenbart eine Spritze zum Einstechen in ein Gewebe mit einer elastisch verformbaren Kanüle. Beide Systeme sind zum Einstechen der Nadel beziehungsweise Kanüle in das intakte Weichgewebe eines Patienten zum Eintragen eines medizinischen Fluids in der Blutkreislauf vorgesehen.

Die lokale Applikation von pharmazeutischen Wirkstoffen wie Antibiotika ist schon seit Jahrzehnten bekannt und bewährt sich insbesondere bei der Behandlung beziehungsweise der Beruhigung von Infektionen des Knochengewebes. Dabei kann man in nicht-resorbierbare und in resorbierbare beziehungsweise biodegradierbare Wirkstoffträger unterscheiden.

Für die Behandlung von Knochenbrüchen sind seit Jahrzehnten eine Vielzahl von erfolgreichen Behandlungsmöglichkeiten, wie beispielweise die Verwendung von Osteosyntheseplatten, Marknägeln und Fixateur externe, etabliert. Man unterscheidet geschlossene und offene Knochenbrüche. Bei geschlossenen Brüchen ist die Fraktur vom Weichgewebe komplett überdeckt. Diese Frakturen sind im allgemeinen gut therapierbar. Dabei kommt es selten Fällen, in ca. 1 Prozent der Fälle, zu Komplikationen durch periimplantäre Infektionen. Im Gegensatz dazu sind bei offenen Frakturen die frakturierten Knochen, infolge von Weichteilverletzungen, nicht oder nur teilweise vom Weichgewebe umschlossen. Die offenen Frakturen sind bis zur medizinischen Versorgung ungeschützt der exogenen Kontamination mit mikrobiellen Keimen ausgesetzt. Mikrobielle Keime können dadurch direkt auf das offene Knochengewebe gelangen. Bei der chirurgischen Behandlung von offenen Frakturen ist es üblich, das freiliegende Knochengewebe intensiv zu reinigen. Dazu werden zum Beispiel Lavage-Systeme eingesetzt, um Kontaminationen des Knochengewebes soweit als möglich durch Spülung zu entfernen. Weiterhin ist eine systemische Antibiotika-Prophylaxe üblich. Leider kommt es trotzdem in ungefähr 25 Prozent der Fälle zu einer Infektion des Knochengewebes durch mikrobielle Keime.

Nach Auftreten einer Früh- oder einer Spätinfektion bei Frakturen wird alles vorhandene Fremdmaterial, wie Osteosyntheseplatten, Schrauben, Pins usw., entfernt und es wird debridiert. Anschließend ist eine systemische Antibiotikatherapie in Kombination mit lokalen Antibiotikaträgern, wie zum Beispiel Gentamicin enthaltende Kollagenschwämme und Polymethylmethacrylat-Ketten, zur Beruhigung der Infektionen üblich, wobei die frakturierten Knochen gleichzeitig durch Osteosynthesematerialien stabilisiert werden. Exemplarisch für resorbierbare beziehungsweise biodegradierbare Wirkstoffträger sind Vliese und Schwämme aus Kollagen oder Gelatine. Exemplarisch seien dafür die Druckschriften DE 34 29 038 A1, DE 33 34 595 A1, DE 28 43 963 C2, DE 32 03 957 C2 und DE 33 34 595 A1 genannt. Diese enthalten Gentamicinsulfat oder Gemische aus Gentamicinsulfat und einem in Wasser gering löslichen Gentamicinsalz. Weiterhin gibt es eine Vielzahl von resorbierbaren beziehungsweise biodegradierbaren Wirkstoffträgern auf Basis von Tricalciumphosphat, Hydroxylapatit, Gips und deren Mischungen sowie auch Kompositmaterialien aus diesen Salzen und organischen Bindemitteln.

Nachteilig an den aufgeführten nicht-resorbierbaren und auch den resorbierbaren beziehungsweise biodegradierbaren Wirkstoffträgern ist, dass der antimikrobielle Wirkstoff durch die gewählte Zusammensetzung festgelegt ist und dass nach der Implantation des Wirkstoffträgers der Wirkstoff nicht mehr ausgetauscht oder mit anderen Wirkstoffen ergänzt werden kann. Weiterhin unterliegt die Wirkstofffreisetzung bei allen bisherigen lokalen Wirkstofffreisetzungssystemen auf dem Prinzip der Diffusion, so dass hohe Wirkstoffmengen nur in den ersten Stunden freigesetzt werden. Eine Ausnahme bildet die Verwendung von in Wasser gering löslichen Wirkstoffsalzen, bei denen die Wirkstofffreisetzung vom Löslichkeitsgleichgewicht der Wirkstoffsalze abhängt.

Periprothetische Infektionen stellen eine sehr ernste Komplikation für die Patienten dar. Diese Infektionen können chronisch werden und sich zur chronischen Osteitis entwickeln, die sehr schwer zu therapieren ist. Daneben ist die Behandlung von periprothetischen Infektionen auch mit einem hohen finanziellen und materiellen Aufwand verbunden.

Wünschenswert ist es daher, eine Besiedlung des frakturierten Knochengewebes bei offenen Frakturen durch mikrobielle Keime so früh wie möglich durch lokales Applizieren von Antibiotika an den potentiell kontaminierten Bruchflächen des Knochengewebes zu verhindern.

Wünschenswert ist dabei ein Wirkstoffträger, der eine lokale Applikation beliebiger pharmazeutischer Wirkstoffe zulässt und wobei der pharmazeutische Wirkstoff jederzeit mit anderen fluiden pharmazeutischen Wirkstoffen ergänzt werden kann. Außerdem ist es wünschenswert, dass die Wirkstoffkonzentration, die unmittelbar an der Fraktur erreicht wird, direkt von außen eingestellt werden kann.

Knochenschrauben mit einem zentralen Kanal im Schraubenkörper und damit verbundenen Fenstern sind unter der Bezeichnung kanellierte und fenestrierte Schrauben bekannt. Bisher werden diese Knochenschrauben im Wesentlichen im Spine-Bereich als sogenannte kanellierte und fenestrierte Pedikelschrauben eingesetzt. Dabei wird durch den Kanal der Schrauben Knochenzementteig in den meist osteoporotischen Wirbelkörper eingespritzt. Es bildet sich meistens ein zur Längsachse der Pedikelschraube koaxialer Zementmantel aus. Der Knochenzement härtet anschließend aus und bildet ein Widerlager für die Pedikelschrauben. Eine Vielzahl von kanellierten und fenestrierten Knochenschrauben wurden vorgeschlagen. Exemplarisch sind dafür die Patenschriften und Offenlegungsschriften DE 35 08 759 A1, RU 2 572 481 C1, RU 2 622 613 C2, US 2001/0021852 A1, US 2005/0015059 A1, US 2012/0029578 A1, US 6 214 012 B1, US 9 326 801 B2, US 8 382 808 B2 und US 8 974 505 B2 genannt.

Eine spezielle kanellierte und fenestrierte Knochenschraube ist in der EP 1 622 529 B1 dargestellt. Dabei wird ein Führungselement temporär in den Kanal der Knochenschraube eingesetzt. Das Führungselement dient dem Einspritzen von Knochenzementteig.

Ein ähnlicher Adapter zum Einschrauben einer kanellierten und fenestrierten Knochenschraube wird in der US 6 048 343 A offenbart. Der Adapter hat einen Längskanal und dichtet mit seiner Außenwand die proximale Bohrung der Knochenschraube ab, so dass Flüssigkeiten in die Knochenschraube durch den Adapter eingepresst werden können. Der Adapter wird nach dem Einpressen der Flüssigkeit in den Knochen entfernt.

Eine in der US 10 188 442 B2 beschriebene Schraube ist für die Tumorbehandlung bestimmt und hat drei oder mehrere Längskanäle im Schraubenkörper, die fenestriert sind. Diese Längskanäle sind zum Anschluss an Katheter vorgesehen. Mit den angeschlossenen Kathetern können flüssige Wirkstoffe in den die Schraube umgebenden Knochen appliziert werden.

Eine mit durchgehenden Längsschlitzen versehene Knochenschraube, bei der auch der Schraubenkopf Längsschlitze aufweist wird mit der EP 2 887 899 B1 vorgeschlagen. Die Längsschlitze des Schraubenkörpers sollen eine bessere Freisetzung von Stoffen, wie Knochenzement, aus der Knochenschraube ermöglichen.

Im Patent EP 0 305 417 B1 wird eine Knochenschraube beschrieben, die kanelliert und fenestriert ist. Diese Knochenschraube wird vakuumdicht im Knochen verschraubt. Es wird ein Saug-Spülsystem mit dieser Knochenschraube beschrieben. Dabei wird bei einer kanellierten und fenestrierten Knochenschraube eine Spülflüssigkeit eingeleitet und an einer zweiten kanellierten und fenestrierten Knochenschraube mit Vakuum die Spülflüssigkeit abgesaugt.

In der US 5 601 559 A1 wird eine kanellierte und fenestrierte Knochenschraube beschrieben, die eine systemischen Applikation von pharmazeutischen Wirkstoffen über das Knochengewebe anstelle von einem venösen Zugang ermöglichen soll.

Eine selbstschneidende Knochenschraube mit Längsschlitzen und einem zentralen Kanal wird in der EP 0 595 782 B1 offenbart. Die Schlitze und der Kanal sollen zuerst das bei dem Einschneiden der Schraube geschnittene Knochenmaterial aufnehmen und dann ein Durchwachsen des Knochengewebes mit der Knochenschraube ermöglichen. Ein Schlitz, in dem mehrere Öffnungen zum inneren Kanal angeordnet sind, erstreckt sich dabei in das Gewinde aber nicht bis zum Schraubenkopf. Der Kanal und die Schlitze werden beim Einschneiden der Knochenschraube durch Knochenmaterial blockiert und können dann nicht mehr zum Leiten eines pharmazeutischen Fluids verwendet werden.

Die Patente US 10 357 298 B2, US 10 349 993 B2 und US 9 616 205 B2 offenbaren Implantate und dabei eine implantierbare Schraube, die einen Längskanal mit Fenestrierungen enthält. Im Längskanal ist unterhalb eines Schraubenkopfes eine sich in distaler Richtung vergrößernde Verjüngung angeordnet. Unterhalb dieser Verjüngung befindet sich ein Reservoir mit nach Außen führenden Kanälen. Der Durchmesser des Kanals im Schraubenkopf ist gleich dem Durchmesser des Reservoirs. Die Länge der vom Reservoir ausgehenden Kanäle ist größer als der Innendurchmesser dieser Kanäle.

Die aus dem Stand der Technik bekannten fenestrierten Knochenschrauben haben den Nachteil, dass diese zur Behandlung einer einfachen Fraktur nicht zwingend notwendig sind. Durch das Einschrauben der Knochenschrauben wird der angrenzende Knochen beschädigt, wobei in den Fällen, in denen der frakturierte Knochen auch ohne Verschraubung zusammenwachsen kann, kein Nutzen durch die Möglichkeit zur Befestigung mit Knochenschrauben gegeben ist. Des Weiteren müssen die Knochenschrauben im Patienten verbleiben oder in einer Operation aufwendig entfernt werden, wobei bei dieser Operation erneut unerwünschte Infektionen auftreten können.

Der Erfindung liegt die Aufgabe zugrunde, die Nachteile des Stands der Technik zu überwinden. Insbesondere soll eine Vorrichtung zur lokalen Applikation von pharmazeutischen Fluiden, wie zum Beispiel von antibiotischen Lösungen, bereitgestellt werden, mit der eine lokale und temporäre Abgabe des pharmazeutischen Fluids direkt im Bereich des Knochens ermöglicht wird, wie zum Beispiel in infizierten frakturierten aber bereits gerichteten Knochen. Die Vorrichtung soll auch über längere Zeiträume an einem bestimmten Ort zur wiederholten Abgabe des pharmazeutischen Fluids geeignet sein, ohne dass die Vorrichtung hierfür entfernt werden muss. Die Vorrichtung soll kostengünstig zu fertigen sein. Die Behandlung mit der Vorrichtung soll in deren Verlauf anpassbar sein, so dass auf eine Veränderung der Behandlungssituation oder auf einen ausbleibenden Erfolg reagiert werden kann. Die Vorrichtung soll ohne eine aufwendige Operation des Patienten wieder leicht entfernbar sein.

Die Aufgabe der Erfindung besteht somit auch darin, eine Vorrichtung in Form eines lokalen Applikationssystems für pharmazeutische Fluide zu entwickeln, das zur Prävention und auch zur Behandlung von periprothetischen Infektionen bei gerichteten frakturierten Knochen bestimmt ist. Das zu entwickelnde Applikationssystem soll eine lokale Applikation und bevorzugt auch eine Spülung mit pharmazeutischen Fluiden bei zumindest teilweiser Weichteildeckung über einen Zeitraum von mehreren Stunden bis Tagen ermöglichen. Es sollen beliebige antiseptische oder antibiotische Lösungen im Bereich des zu behandelnden Knochens eines Patienten applizierbar sein.

Die Aufgabe der Erfindung besteht insbesondere in der Entwicklung einer Vorrichtung zum temporären lokalen Applizieren von pharmazeutischen Wirkstofflösungen in Knochengewebe, speziell in den Frakturspalt von frakturierten Knochen. Dabei soll die Vorrichtung so beschaffen sein, dass diese direkt beim Reponieren der frakturierten Knochen in den Frakturspalt eingesetzt werden kann und dass ein Zusammenwachsen des Knochengewebes weitgehend ungehindert erfolgen kann. Die Vorrichtung soll eine Wirkstoffapplikation im Inneren von Knochen über einen Zeitraum von mehreren Stunden bis Tagen ermöglichen. Damit soll einer Besiedlung des Knochengewebes mit mikrobiellen Keimen entgegengewirkt werden. Es soll möglich sein, wässrige Wirkstofflösungen beliebiger Zusammensetzung mit der Vorrichtung zu applizieren. Die Vorrichtung soll eine solche Oberfläche besitzen, dass ein Einwachsen von Bindegewebe in die Vorrichtung nicht möglich ist. Nach Beendigung der Wirkstoffapplikation soll die Vorrichtung möglichst einfach und schonend entfernt werden können. Wichtig ist, dass die Vorrichtung so beschaffen ist, dass die Wirkstofflösungen an der festgelegten Position im Knochen appliziert werden können und die Vorrichtung nicht durch unbeabsichtigte äußere Zugbeanspruchung während des Applizierens der Wirkstofflösung aus dieser Position verschoben werden kann.

Die Aufgaben der Erfindung werden gelöst durch eine Vorrichtung zur lokalen Applikation von pharmazeutischen Fluiden, die Vorrichtung aufweisend
mindestens eine Kapillare mit mindestens einer Öffnung im Bereich des distalen Endes der mindestens einen Kapillare,
ein hohles Zwischenstück, das an einer der mindestens einen Öffnung gegenüberliegenden Seite der mindestens einen Kapillare mit der mindestens einen Kapillare flüssigkeitsdurchlässig verbunden ist,
eine Führung zum Führen des Zwischenstücks, wobei die Führung eine distale Auflage zur Auflage auf den Patienten aufweist,
ein Fixierungselement zur Fixierung des Zwischenstücks an der Führung, wobei mit dem Fixierungselement eine Fixierung des Zwischenstücks in verschiedenen Positionen relativ zur Führung möglich ist, wobei die Fixierung eine Verschiebung des Zwischenstücks gegen die Führung verhindert und wobei das Fixierungselement eine an der Führung angeordnete Haltevorrichtung oder Klemmvorrichtung ist, oder das Fixierungselement eine Schraube oder eine Flügelschraube ist.

Unter dem Begriff "Kapillare" wird erfindungsgemäß ein hohles Rohr mit einem Außendurchmesser von maximal 3 mm, bevorzugt von maximal 2 mm und besonders bevorzugt von maximal 1 mm verstanden.

Die distale Auflage kann erfindungsgemäß durch eine distale Auflagefläche zur Auflage auf eine Patientenoberfläche realisiert werden. Bevorzugt ist die Auflage eine durchbrochene Scheibe, die ein distales Ende der Führung bildet. Dadurch besitzt die Führung eine gute Auflage auf der Körperoberfläche des Patienten. Diese Scheibe kann in einfacher weise mit Pflastern auf der Hautoberfläche befestigt werden oder auch auf die Haut aufgenäht werden.

Es kann erfindungsgemäß vorgesehen sein, dass in einem Hohlraum der mindestens einen Kapillare und des Zwischenstücks ein Kern angeordnet ist, wobei der Kern aus dem Hohlraum der mindestens einen Kapillare entfernbar ist, wobei der Kern mindestens 50 % der Querschnittsfläche der mindestens einen Öffnung verschließt, und wobei der Kern mit dem Zwischenstück derart lösbar verbunden ist, dass die Verbindung eine axiale Bewegung des Kerns im Hohlraum der mindestens einen Kapillare einschränkt oder verhindert.

Es kann vorgesehen sein, dass der Kern zylindrisch geformt ist.

Es kann auch vorgesehen sein, dass der Kern am Zwischenstück lösbar verankert ist.

Bevorzugt kann vorgesehen sein, dass der Kern aus dem Hohlraum der mindestens einen Kapillare und dem Zwischenstück manuell entfernbar ist. Es kann theoretisch ausreichen, wenn der Kern aus der mindestens einen Kapillare entfernbar ist und der die mindestens eine Kapillare zuvor verschließende Teil des Kerns in dem Hohlraum des Zwischenstücks verbleibt. Der Hohlraum des Zwischenstücks weist einen größeren Innendurchmesser als die mindestens eine Kapillare auf, so dass das pharmazeutische Fluid durch den Hohlraum des Zwischenstücks an dem die mindestens eine Kapillare zuvor verschließenden Teil des Kerns, der nun im Hohlraum des Zwischenstücks angeordnet ist, problemlos vorbeifließen kann.

Es kann vorgesehen sein, dass der Kern ein dünner Draht oder Faden zum Verschließen der mindestens einen Öffnung einer einzelnen Kapillare ist oder dass der Kern ein Drahtbündel dünner Drähte mit vereinzelten Enden oder ein Fadenbündel dünner Fäden mit vereinzelten Enden zum Verschließen der mindestens einen Öffnung mehrerer Kapillaren ist. Hiermit kann der Kern leichter aus den Kapillaren und dem Zwischenstück gezogen werden.

Es kann ferner vorgesehen sein, dass der Kern an wenigstens einem distalen Ende eine Verdickung zum Verschließen der mindestens einen Öffnung der mindestens einen Kapillare aufweist.

Es kann auch vorgesehen sein, dass der Kern mindestens 50 % der Querschnittsfläche des Hohlraums der mindestens einen Kapillare bis zur mindestens einen Öffnung verschließt, insbesondere bis zur der am dichtesten zum distalen Ende der mindestens einen Kapillare angeordneten Öffnung der mindestens einen Öffnung verschließt.

Des Weiteren kann vorgesehen sein, dass das Fixierungselement eine mechanische Fixierung des Zwischenstücks an der Führung ermöglicht. Alternativ wäre beispielsweise auch eine magnetische Fixierung denkbar.

Bevorzugt kann auch vorgesehen sein, dass durch die Fixierung des Zwischenstücks an der Führung eine axiale Bewegung des Kerns gegen den Hohlraum der mindestens einen Kapillare während einer Verformung der mindestens einen Kapillare ausgeschlossen ist und bevorzugt auch während der Verformung des Zwischenstücks ausgeschlossen ist.

Es kann auch vorgesehen sein, dass durch die Fixierung des Zwischenstücks an der Führung eine axiale Bewegung des Kerns gegen den Hohlraum der mindestens einen Kapillare während der Implantation ausgeschlossen ist.

Es kann im Rahmen der vorliegenden Erfindung vorgesehen sein, dass der Kern durch einen Adapter aus dem Hohlraum der mindestens einen Kapillare ziehbar ist und bevorzugt auch aus dem Hohlraum des hohlen Zwischenstücks ziehbar ist.

Hierdurch kann der Kern nach der Implantation aus der mindestens einen Kapillare gezogen werden, um die Durchleitung eines pharmazeutischen Fluids zu ermöglichen. Der Adapter ist vorzugsweise am proximalen Ende des Zwischenstücks angeordnet, insbesondere befestigt. Der Adapter kann dann vorzugsweise auch zum Anschließen einer Quelle für das pharmazeutische Fluid verwendet werden, besonders bevorzugt zum Herstellen einer fluiddichten Verbindung. Die Quelle für das pharmazeutische Fluid kann insbesondere eine Spritze sein.

Ferner kann vorgesehen sein, dass die Vorrichtung einen Schlauch aufweist, der mit dem Zwischenstück flüssigkeitsdurchlässig verbunden oder verbindbar ist, wobei der Schlauch verformbar ist.

Hierdurch kann die Vorrichtung von der Quelle des pharmazeutischen Fluids durch den Schlauch getrennt werden, so dass bei einer Bewegung der Quelle keine mechanische Belastung der Vorrichtung auftritt. Als Quelle kann vorzugsweise eine Spritze verwendet werden. Der Schlauch ermöglicht es auch, die Quelle separat von der Vorrichtung auszustellen.

Der verformbare Schlauch ist vorzugsweise verformbar.

Es kann auch vorgesehen sein, dass das Zwischenstück, insbesondere über den Schlauch zuvor erwähnten Schlauch, mit einem Fluidreservoir flüssigkeitsdurchlässig verbunden oder verbindbar ist, wobei ein pharmazeutisches Fluid aus dem Fluidreservoir unter Druck durch das Zwischenstück und durch die mindestens eine Kapillare, und sofern vorhanden auch durch den Schlauch, aus der mindestens einen Öffnung der mindestens einen Kapillare pressbar ist.

Hierdurch kann ein unmittelbares Durchleiten des pharmazeutischen Fluids durch die Vorrichtung erfolgen.

Des Weiteren kann vorgesehen sein, dass das Zwischenstück hohlzylinderförmig oder schlauchförmig ist, wobei vorzugsweise das Zwischenstück an seiner mit der mindestens einen Kapillare verbundenen distalen Seite eine Verjüngung aufweist, bevorzugt eine konische Verjüngung, wobei besonders bevorzugt die konische Verjüngung ein hohler Konus ist.

Hierdurch ist der Aufbau besonders kostengünstig zu realisieren und das pharmazeutische Fluid kann leicht durch das Zwischenstück in die mindestens eine Kapillare fließen.

Ferner kann vorgesehen sein, dass das Zwischenstück in der Führung axial beweglich angeordnet ist, wenn die Fixierung des Fixierungselements gelöst ist.

Hiermit kann das Zwischenstück bei gelöster Fixierung geführt von der Führung in der Führung bewegt werden und dadurch die mindestens eine Kapillare mit der Vorrichtung positioniert werden.

Es kann vorgesehen sein, dass die Führung hohlzylinderförmig oder schlauchförmig ist.

Dadurch kann das Zwischenstück mit der mindestens einen Kapillare ohne großen Widerstand in der Führung geführt werden.

Gemäß einer bevorzugten Weiterbildung der vorliegenden Erfindung kann vorgesehen sein, dass der Kern mit einem Gewinde oder einem Bajonettverschluss mit dem Zwischenstück lösbar verbunden ist.

Hierdurch wird eine stabile aber auch einfach zu lösende Verbindung zwischen dem Kern und dem Zwischenstück bereitgestellt. Der Anwender kann diese Verbindung ohne Probleme lösen.

Es ist vorgesehen, dass das Fixierungselement eine an der Führung angeordnete Haltevorrichtung oder Klemmvorrichtung ist, insbesondere zusammenschraubbare Klemmbacken sind, oder das Fixierungselement eine Schraube ist, insbesondere eine Flügelschraube ist, wobei bevorzugt die Schraube in einem durch eine Wandung der Führung reichende Bohrung mit einem Innengewinde eingeschraubt oder einschraubbar ist, so dass mit einem vorderen Ende der Schraube das Zwischenstück in der Führung einklemmbar ist.

Hierdurch kann das Zwischenstück auf einfache Weise in jeder Lage relativ zur Führung fixiert werden. Theoretisch kann auch eine Klemmvorrichtung und eine Schraube gemeinsam als doppeltes Fixierungselement verwendet werden.

Alternativ zur Schraube kann zur mechanischen Fixierung des Zwischenstücks eine Klemmvorrichtung an der Führung angeordnet sein. Im Rahmen der Erfindung können zur Fixierung des Zwischenstücks Rastvorrichtungen oder auch lösbare Klettverbindungen oder auch lösbare Klebverbindungen verwendet werden.

Es kann vorgesehen sein, dass das Zwischenstück mit dem Fixierungselement in einer beliebigen axialen Lage an der Führung einklemmbar ist. Dadurch kann das Zwischenstück in beliebigen Positionen an der Führung fixiert werden. Hierdurch wiederum kann genau eingestellt werden, wie weit die mindestens eine Kapillare aus der Führung herausragt und damit, in welcher Tiefe im Patienten, insbesondere im Knochen des Patienten das oder die distalen Ende(n) der mindestens einen Kapillare angeordnet werden.

Des Weiteren kann vorgesehen sein, dass das Zwischenstück für Flüssigkeiten durchlässig ist, wenn das Zwischenstück mit dem Fixierungselement in der Führung fixiert ist.

Hierdurch kann sichergestellt werden, dass das pharmazeutische Fluid durch das fixierte Zwischenstück in die mindestens eine Kapillare gepresst werden kann. Das bedeutet, dass die Fixierung durch das Fixierungselement nicht den Hohlraum des Zwischenstücks verschließt.

Auch kann vorgesehen sein, dass der Hohlraum der Führung mindesten so lang ist, wie die Länge der mindestens einen Kapillare beziehungsweise wie die Länge der längsten Kapillare der mindestens einen Kapillare.

Hierdurch wird erreicht, dass die mindestens eine Kapillare vollständig in der Führung versenkt werden kann und damit dort sicher aufbewahrt werden kann.

Bevorzugt kann des Weiteren vorgesehen sein, dass der Hohlraum in der Führung am distalen Ende zulaufend ist, insbesondere stetig zulaufend oder konisch zulaufend ist, und dass eine Durchbrechung am distalen Ende des zulaufenden Bereichs, insbesondere an einer Spitze eines Konus angeordnet ist, durch den die mindestens eine Kapillare durch das distale Ende der Führung hindurchgeführt ist oder hindurchführbar ist, wobei bevorzugt durch Durchmesser der Durchbrechung kleiner ist als der Außendurchmesser des Zwischenstücks.

Hierdurch kann die mindestens eine Kapillare aus der Führung hervorgeschoben und dabei an der Führung gehalten werden. Durch die insbesondere konische Verjüngung lässt sich die mindestens eine Kapillare problemlos bei der Montage der Vorrichtung in die Führung einsetzen.

Mit der vorliegenden Erfindung wird auch vorgeschlagen, dass die Vorrichtung einen Behälter für das pharmazeutische Fluid aufweist, wobei bevorzugt in dem Behälter ein pharmazeutisches Fluid enthalten ist, besonders bevorzugt enthaltend zumindest einen antibiotischen Wirkstoff, zumindest einen antimykotischen Wirkstoff und/oder zumindest ein Chemotherapeutikum.

Hierdurch wird die Vorrichtung weiter komplettiert und kann unmittelbar zur Behandlung eingesetzt werden.

Es kann dabei vorgesehen sein, dass der Behälter einen Hohlzylinder mit einem axial im Hohlzylinder verschiebbaren Kolben aufweist, der ein erstes Ende des Hohlzylinders verschließt, wobei der Hohlzylinder an einem dem ersten Ende gegenüberliegenden Ende eine Austragsöffnung aufweist, die mit einer proximalen Seite des Zwischenstücks verbunden oder verbindbar ist, bevorzugt über ein manuell bedienbares Ventilelement zur Regulierung der Strömungsgeschwindigkeit des pharmazeutischen Fluids mit der proximalen Seite des Zwischenstücks verbunden oder verbindbar ist.

Der Behälter kann über den Schlauch mit der proximalen Seite des Zwischenstücks verbunden sein. Der Schlauch kann also zwischen dem Behälter und dem Zwischenstück angeordnet sein. Die proximale Seite des Zwischenstücks liegt der distalen Seite des Zwischenstücks gegenüber, an der die mindestens eine Kapillare angeordnet ist.

Es kann auch vorgesehen sein, dass die Vorrichtung eine Fördereinrichtung aufweist, mit der das pharmazeutische Fluid aus einem mit der Fördereinrichtung verbundenen oder verbindbaren Behälter in das Zwischenstück und durch die mindestens eine Kapillare und durch die mindestens eine Öffnung in die Umgebung der mindestens einen Kapillare drückbar ist, wobei vorzugsweise die Fördereinrichtung ein Energiespeicherelement, insbesondere zumindest eine gespannte Feder, aufweist, wobei die Fördereinrichtung mit Energie aus dem Energiespeicherelement antreibbar ist, wobei besonders bevorzugt mit dem Energiespeicherelement ein Kolben in einem Hohlzylinder als der Behälter in Richtung einer gegenüberliegenden Austragsöffnung anzutreiben ist.

Mit einer Fördereinrichtung kann die Vorrichtung direkt zum Erzeugen eines Volumenstroms des pharmazeutischen Fluids verwendet werden. Wenn die Fördereinrichtung ein Energiespeicherelement umfasst, muss die Vorrichtung nicht an eine externe Energieversorgung zum Antreiben der Fördereinrichtung angeschlossen werden. Eine gespannte Feder enthält dabei genug Energie, um eine Menge von einigen Millilitern bis einigen Zentilitern des pharmazeutischen Fluids mit der Vorrichtung auszupressen.

Des Weiteren kann vorgesehen sein, dass der Kern über wenigstens die am dichtesten am distalen Ende der mindestens einen Kapillare angeordnete Öffnung soweit heraussteht, dass bei einer Biegung der mindestens einen Kapillare diese Öffnung zu mindestens 50 % der Querschnittsfläche dieser Öffnung verschlossen ist.

Hierdurch wird sichergestellt, dass die mindestens eine Öffnung der mindestens eine Kapillare auch beim Biegen der mindestens einen Kapillare verschlossen bleibt. Hierdurch wird verhindert, dass Gewebereste oder andere Verunreinigungen in die mindestens eine Öffnung beziehungsweise in die mindestens eine Kapillare eindringen können und diese verstopfen. Dadurch wird auch bei einer Biegung der mindestens einen Kapillare verhindert, dass während der Implantation Gewebebestandteile die mindestens eine Kapillare verstopfen. Vorteilhaft ist es, wenn der Kern ungefähr 3 mm in Bezug zur mindestens einen Öffnung der mindestens einen Kapillare übersteht.

Gemäß einer Weiterbildung der vorliegenden Erfindung kann vorgesehen sein, dass die mindestens eine Kapillare mindestens zwei Kapillaren sind, wobei die mindestens zwei Kapillaren eine unterschiedliche Länge haben, bevorzugt alle Kapillaren der mindestens zwei Kapillaren eine unterschiedliche Länge haben.

Hierdurch kann eine Behandlung in unterschiedlichen Tiefen mit der gleichen Freigabe des pharmazeutischen Fluids erfolgen. Vorteilhaft ist es dabei, dass die mit dem Zwischenstück verbundenen mindestens zwei flüssigkeitsdurchlässigen Kapillaren eine unterschiedliche Länge besitzen. Dadurch ist es möglich, verschiedene Bereiche des Frakturspalts lokal mit Wirkstofflösungen zu versorgen.

Bevorzugt kann auch vorgesehen sein, dass die mindestens eine Kapillare aus Titan, aus Edelstahl oder aus Kunststoff besteht, insbesondere aus einem formstabilen Kunststoff besteht.

Diese Materialien sind biokompatibel und gut zur Herstellung der mindestens einen Kapillare geeignet. Daneben können auch alle sonstigen biokompatiblen Metalllegierungen verwendet werden. Möglich ist auch die Verwendung von hochfesten Glaskapillaren oder Keramikkapillaren, die dann aber nicht oder kaum verformbar sind.

Des Weiteren kann vorgesehen sein, dass am distalen Ende der Führung eine poröse schwammartige Scheibe angeordnet ist, wobei vorzugsweise die poröse schwammartige Scheibe eine antiseptische Flüssigkeit, insbesondere eine antiseptische Lösung enthält.

Hierdurch wird die Umgebung des Eintrittskanals in den Körper des Patienten bei der medizinischen Anwendung der Vorrichtung keimfrei oder keimreduziert gehalten. Hierdurch kann der Eintritt von Keimen in den Kanal, in dem die mindestens eine Kapillare angeordnet wird, verhindert oder verringert werden. Dadurch wird einer zusätzlichen Infektion vorgebeugt. Durch die antiseptisch geladene poröse schwammartige Scheibe wird eine Einwanderung von mikrobiellen Keimen entlang der mindestens einen Kapillare in das Weich- und Knochengewebe verhindert.

Auch kann vorgesehen sein, dass die mindestens eine Kapillare und der Kern verformbar sind, wobei vorzugsweise die mindestens eine Kapillare plastisch verformbar ist.

Hierdurch kann die Form der mindestens einen Kapillare and die Behandlungssituation angepasst werden. Zudem kann auch die Führung gebogen sein oder werden.

Dass die mindestens eine Kapillare verformbar ist, bedeutet, dass die mindestens eine Kapillare in beschränktem Umfang zerstörungsfrei verformt werden kann. In beschränktem Umfang bedeutet dabei, dass die mindestens eine Kapillare gebogen aber nicht geknickt werden darf. Für den Kern gilt das analog.

Vorzugsweise kann also vorgesehen sein, dass die mindestens eine Kapillare durch Biegen (aber nicht durch Knicken) zerstörungsfrei verformbar ist. Ein Knicken kann besonders bevorzugt vorliegen, wenn ein Krümmungsradius von 0,1 mm unterschritten wird, ganz besonders bevorzugt wenn ein Krümmungsradius von 0,5 mm unterschritten wird.

Die mindestens eine Kapillare ist bevorzugt plastisch und/oder elastisch verformbar.

Bevorzugt ist die mindestens eine Kapillare in axialer Richtung verformbar und besonders bevorzugt in radialer Richtung nicht oder nur bei unsachgemäßer Handhabung verformbar. Die axiale Richtung bezieht sich dabei auf die Leitungsrichtung der mindestens einen Kapillare.

Die der vorliegenden Erfindung zugrundeliegenden Aufgaben werden auch gelöst durch ein Verfahren zur Vorbereitung einer erfindungsgemäßen Vorrichtung zur lokalen Applikation von pharmazeutischen Fluiden vor einer medizinischen Anwendung mit den folgenden chronologisch nacheinander ablaufenden Schritten:
A) Herausdrücken der mindestens einen Kapillare aus der Führung durch Einschieben des Zwischenstücks in die Führung; und
B) Fixieren des Zwischenstücks in der Führung mit dem Fixierungselement.

Das Verfahren wird nicht zur Behandlung des menschlichen oder eines tierischen Körpers eingesetzt. Eine medizinische Behandlung erfolgt erst durch die Zugabe und Abgabe des pharmazeutischen Fluids, die nicht Teil des erfindungsgemäßen Verfahrens sind.

Gemäß einer bevorzugten Weiterbildung der vorliegenden Erfindung kann vorgesehen sein, dass ein in der mindestens einen Kapillare angeordneter Kern in Schritt A), und vorzugsweise auch in Schritt B), in der mindestens einen Kapillare angeordnet bleibt.

Bei dem erfindungsgemäßen Verfahren findet also keine von der Patentierung ausgeschlossene medizinische Behandlung eines menschlichen oder eines tierischen Körpers statt.

Dabei kann vorgesehen sein, dass in einem Schritt C) nach Schritt B) oder nach Schritt A) ein Umformen der mindestens einen Kapillare durch eine plastische Verformung der mindestens einen Kapillare erfolgt.

Es kann auch vorgesehen sein, dass eine Quelle für ein pharmazeutisches Fluid oder ein Behälter enthaltend ein pharmazeutisches Fluid an eine proximale Seite des Zwischenstücks angeschlossen wird, so dass eine fluidleitende und/oder fluiddichte Verbindung zwischen der Quelle oder dem Behälter und dem Hohlraum der mindestens einen Kapillare entsteht.

Es kann erfindungsgemäß auch vorgesehen sein, dass am distalen Ende der Führung eine poröse schwammartige Scheibe angeordnet wird oder ist, wobei die poröse schwammartige Scheibe mit einer antiseptischen Flüssigkeit, insbesondere einer antiseptischen Lösung, getränkt oder beladen wird.

Vor dem Einsetzen der Vorrichtung kann der Kern aus dem Hohlraum der mindestens einen Kapillare entfernt werden. Bevorzugt erfolgt dieser Schritt als letzter Schritt vor der medizinischen Anwendung der Vorrichtung oder nach dem Einsetzen beim Patienten, um eine Verstopfung der mindestens einen Öffnung der mindestens einen Kapillare zu verhindern.

Ferner kann zur Behandlung die distale Auflage am Patienten befestigt werden, insbesondere auf die Haut des Patienten geklebt werden.

Der vorliegenden Erfindung liegt die überraschende Erkenntnis zugrunde, dass es durch zumindest eine geführte Kapillare, die bevorzugt mit einem herausnehmbaren Kern verschlossen ist, und die gegen eine am Patienten anzuordnende Führung arretierbar ist, gelingt, eine Vorrichtung bereitzustellen, mit der auf sichere Weise eine lokale Applikation von pharmazeutischen Fluiden in den Verbindungsstellen von frakturierten Knochen ermöglicht wird. Bevorzugt sind die Kapillaren durch den herausnehmbaren Kern vor Verstopfung gesichert. Die zumindest eine Kapillare kann nach erfolgter Behandlung schonend aus dem im Zusammenwachsen begriffenen Knochen wieder herausgezogen werden, ohne eine starke mechanische Belastung der heilenden Fraktur zu bewirken. Gleichzeitig können in der Fraktur auch während des Heilungsprozesses pharmazeutische Fluide zur Behandlung der Fraktur abgegeben werden und damit der Heilungserfolg verbessert werden. Zudem ist eine Anpassung der Behandlung während der Behandlung durch die Zugabe veränderter pharmazeutischer Fluide möglich.

Die Vorrichtung wird in der Weise verwendet, dass zuerst die gewünschte Position der mindestens einen Öffnung der zumindest einen Kapillare im Frakturspalt bestimmt wird, an der die Wirkstofflösung appliziert werden soll. Dann wird das Zwischenstück soweit in die Führung geschoben, bis die mindestens eine Kapillare soweit aus der Führung ragt, dass beim Aufsetzten der Führung auf die Körperoberfläche des Patienten die mindestens eine Kapillare genau die vorgesehene Position im Frakturspalt erreichen kann. Dann wird das Zwischenstück an der Führung mechanisch fixiert. Dadurch wird die Länge der mindestens einen Kapillare fixiert. Wenn zwei oder mehrere Kapillaren verwendet werden, dann werden diese so gebogen, dass der Querschnitt der Bruchfläche ausreichend überdeckt beziehungsweise abgedeckt wird. Die dünnen Kapillaren behindern im Bruchspalt nur geringfügig das Zusammenwachsen des Knochengewebes. Es kann bei nicht beanspruchten medizinischen Verfahren eine minimale Nut in die Kortikalis eingebracht werden, damit die eine Kapillare beziehungsweise die mehreren Kapillaren in den Bruchspalt gelegt werden können, ohne dass die Frakturenden voneinander beabstandet sind. Bevorzugt erst nach erfolgtem Reponieren wird bei nicht beanspruchten medizinischen Verfahren anschließend der Kern vom Zwischenstück gelöst und herausgezogen, so dass der Hohlraum der Kapillare oder die Hohlräume der Kapillaren frei sind. Danach kann wird das Zwischenstück mit einem Schlauch oder direkt mit einer Quelle für das pharmazeutische Fluid verbunden. In einfacher Weise können durch Verwendung von üblichen Spritzen wässrige Wirkstofflösungen in die Vorrichtung injiziert werden. Die Wirkstofflösung fließt dann durch den Schlauch über das Zwischenstück in die mindestens eine Kapillare und tritt dann aus der distalen Öffnung oder den distalen Öffnungen aus. Dadurch ist es möglich, direkt im Frakturspalt lokal sehr hohe Wirkstoffkonzentrationen zu erreichen, die zur Behandlung der Fraktur verwendet werden. Nach einer vorgegebenen Zeit von Stunden bis Tagen wird die eine Kapillare oder werden die Kapillaren einfach aus dem Frakturspalt herausgezogen.

Eine beispielhafte erfindungsgemäße Vorrichtung kann zusammengesetzt sein aus
a) mindestens einer plastisch verformbaren Kapillare mit mindestens einer Öffnung im Bereich des distalen Endes der Kapillare,
b) einem hohlen Zwischenstück, das mit der mindestens einen Kapillare flüssigkeitsdurchlässig verbunden ist,
c) einem elastischen Schlauch, der mit dem Zwischenstück flüssigkeitsdurchlässig verbunden oder verbindbar ist,
d) einer Führung für das Zwischenstück, die eine distale Auflagefläche zur Auflage auf die Patientenoberfläche besitzt,
e) einem Fixierungselement zur mechanischen Fixierung des Zwischenstücks an der Führung, wodurch eine Relativbewegung des Zwischenstücks gegen die Führung verhindert wird,
f) wobei im Hohlraum der mindestens einen Kapillare und des Zwischenstücks vor der Implantation ein manuell zu entfernender, plastisch oder elastisch verformbarer zylindrischer Kern angeordnet ist, der mindestens 50 % der Querschnittsfläche der mindestens einen Öffnung der mindestens einen Kapillare verschließt, wobei nach der Implantation der Kern durch einen Adapter aus dem Hohlraum der mindestens einen Kapillare gezogen wird, und wobei
g) der Kern am Zwischenstück lösbar so verankert ist, dass eine axiale Bewegung des Kerns während der Implantation ausgeschlossen ist.

Eine weitere beispielhafte erfindungsgemäße Vorrichtung kann zusammengesetzt sein aus
a) mindestens einer plastisch verformbaren Kapillare mit mindestens einer Öffnung im Bereich des distalen Endes der mindestens einen Kapillare,
b) einem hohlzylinderförmigen Zwischenstück, das mit der mindestens einen Kapillare flüssigkeitsdurchlässig verbunden ist,
c) einem elastischen Schlauch, der mit dem Zwischenstück flüssigkeitsdurchlässig verbunden oder verbindbar ist,
d) einer hohlzylinderförmigen Führung, die eine distale Auflagefläche zur Auflage auf die Patientenoberfläche besitzt, wobei in der hohlzylinderförmigen Führung das hohlzylinderförmige Zwischenstück axial beweglich angeordnet ist,
e) einem Fixierelement zur mechanischen Fixierung des Zwischenstücks an der Führung, wodurch eine Relativbewegung des hohlzylinderförmigen Zwischenstücks gegen die Führung verhindert wird,
f) wobei im Hohlraum der mindestens einen Kapillare und des Zwischenstücks vor der Implantation ein manuell zu entfernender, plastisch oder elastisch verformbarer zylindrischer Kern angeordnet ist, der mindestens 50 % der Querschnittsfläche der mindestens einen Öffnung der mindestens einen Kapillare verschließt, wobei nach der Implantation der Kern durch einen Adapter aus dem Hohlraum der mindestens einen Kapillare gezogen wird, und wobei
g) der Kern am hohlzylinderförmigen Zwischenstück mit einem Gewinde oder einem Bajonett-Verschluss lösbar so verankert ist, dass eine axiale Bewegung des Kerns während der Implantation ausgeschlossen ist.

Im Folgenden werden weitere Ausführungsbeispiele der Erfindung anhand von fünfzehn schematisch dargestellten Figuren erläutert, ohne jedoch dabei die Erfindung zu beschränken.

Dabei zeigt:
Figur 1: eine schematische Querschnittansicht durch eine erste erfindungsgemäße Vorrichtung zur lokalen Applikation von pharmazeutischen Fluiden;
Figur 2: eine schematische Seitenansicht der Vorrichtung nach Figur 1 mit gerader Führung sowie mit einer Spritze und einem Adapter zum Einpressen des Fluids in die Vorrichtung;
Figur 3: eine schematische Seitenansicht der Vorrichtung nach Figur 1 mit ausgefahrener Kapillare;
Figur 4: eine schematische Querschnittansicht der ersten erfindungsgemäßen Vorrichtung nach den Figuren 1 bis 3 mit herausgezogenem Kern;
Figur 5: eine schematische Seitenansicht der ersten erfindungsgemäßen Vorrichtung nach den Figuren 1 bis 3 mit herausgezogenem Kern;
Figur 6: eine schematische Querschnittansicht der ersten erfindungsgemäßen Vorrichtung mit über einen Schlauch angeschlossener Spritze;
Figur 7: eine schematische Seitenansicht der ersten erfindungsgemäßen Vorrichtung mit angeschlossener Spritze;
Figur 8: eine schematische Querschnittansicht der ersten erfindungsgemäßen Vorrichtung mit über einen Schlauch angeschlossener und ausgepresster Spritze;
Figur 9: eine schematische Querschnittansicht der ersten erfindungsgemäßen Vorrichtung während der Behandlung mit über einen Schlauch angeschlossener und ausgepresster Spritze;
Figur 10: eine Ausschnittvergrößerung der Figur 9;
Figur 11: eine schematische Querschnittansicht einer zweiten erfindungsgemäßen Vorrichtung während der Behandlung mit über einen Schlauch angeschlossener und ausgepresster Spritze;
Figur 12: eine Ausschnittvergrößerung der Figur 1;
Figur 13: eine Ausschnittvergrößerung der Figur 4;
Figur 14: eine Ausschnittvergrößerung der Figur 9; und
Figur 15: eine Ausschnittvergrößerung der Figur 11.

In den Figuren und in der folgenden Beschreibung zu den anhand der Figuren erläuterten Ausführungsbeispielen der vorliegenden Erfindung werden teilweise für unterschiedliche Ausführungsbeispiele und für unterschiedliche Einzelteile die gleichen Bezugszeichen bei gleichen oder gleichartigen Teilen verwendet, um die Vergleichbarkeit der Ausführungsbeispiele und die Lesbarkeit zu vereinfachen.

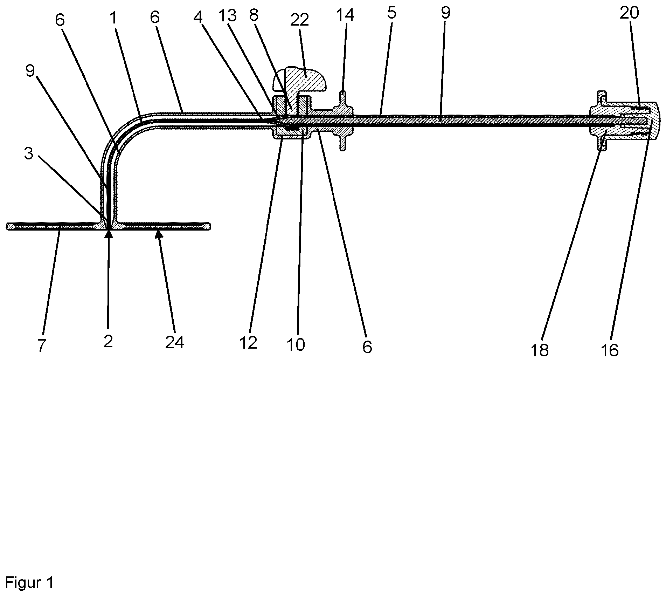

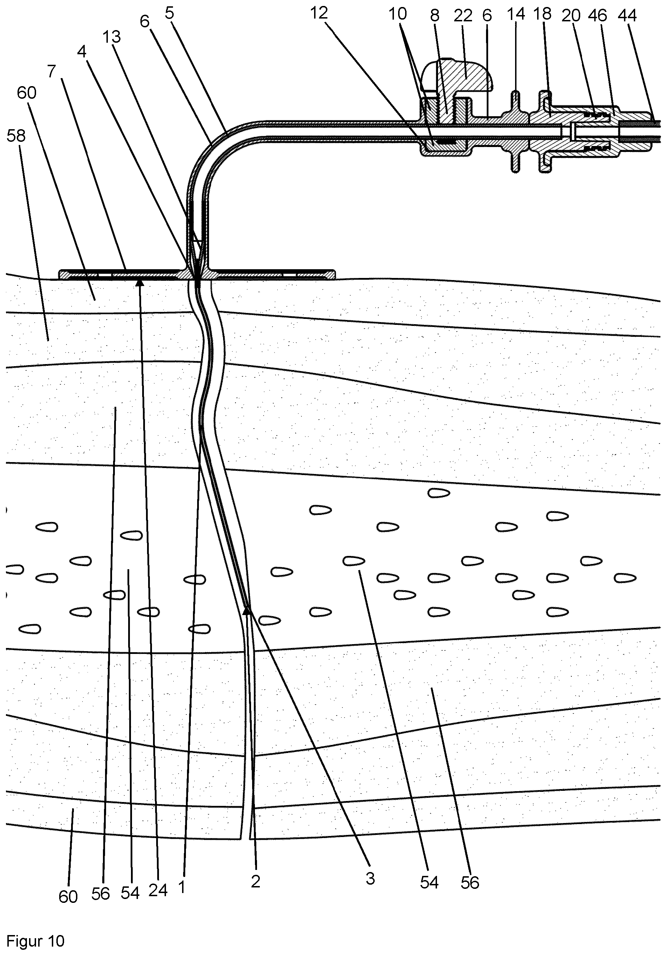

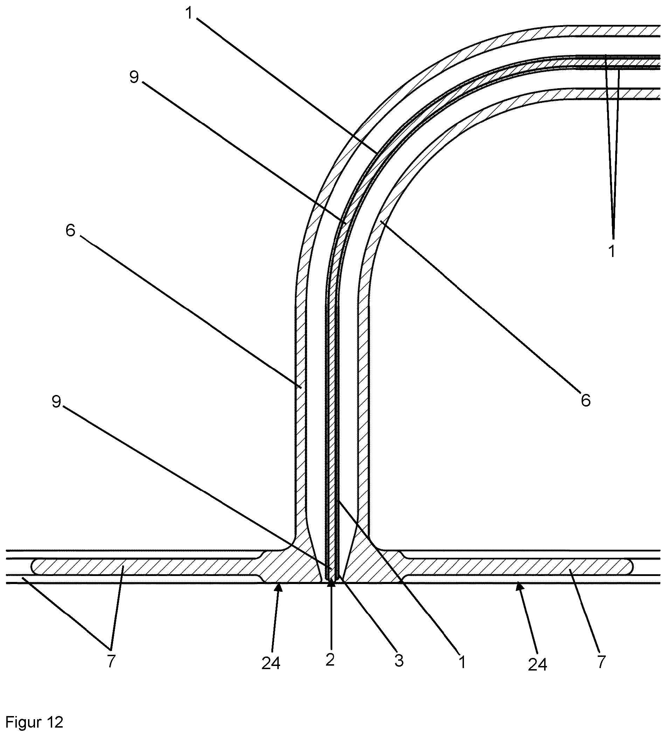

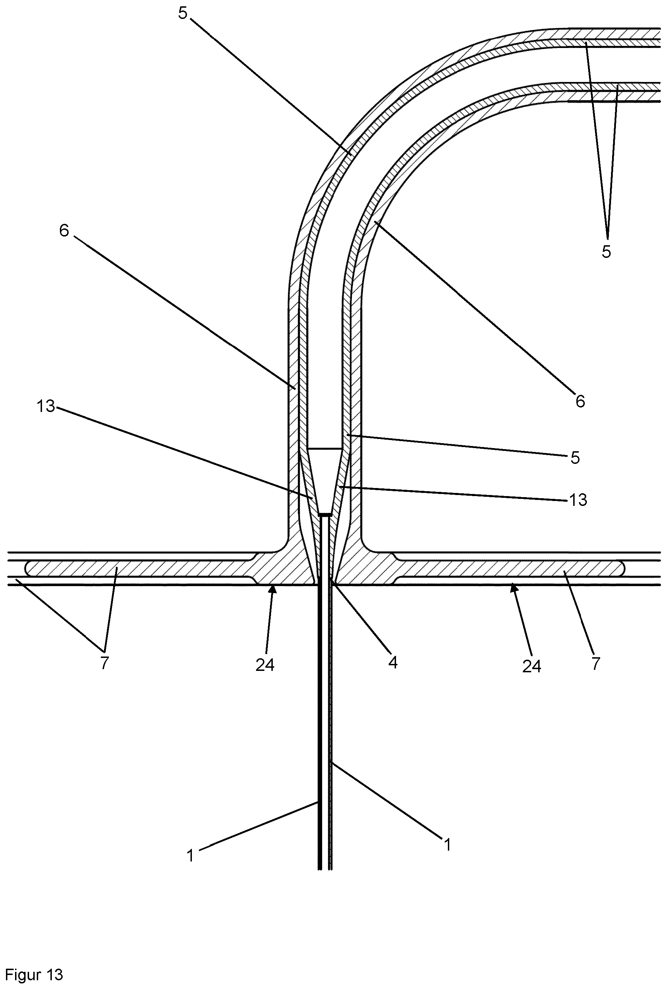

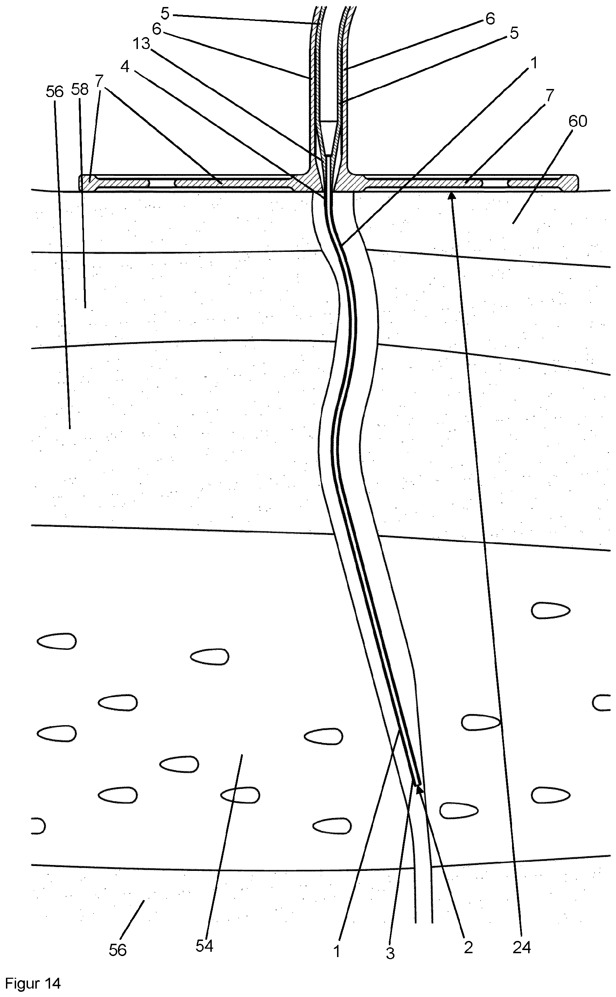

Die Figuren 1 bis 10 und 12 bis 14 zeigen eine erste erfindungsgemäße Vorrichtung in unterschiedlichen Darstellungen und in unterschiedlichen Situationen. Die Vorrichtung weist eine Kapillare 1 auf. Die Kapillare 1 kann eine Öffnung 2 in einem distalen Ende 3 der Kapillare 1 aufweisen. Es können aber auch mehrere Öffnungen (nicht gezeigt) in der Wandung der Kapillare 1 vorgesehen sein.

An einem dem distalen Ende 3 gegenüberliegenden proximalen Ende 4 der Kapillare 1 kann die Kapillare 1 mit einem hohlen Zwischenstück 5 verbunden sein. Die Kapillare 1 begrenzt einen Hohlraum in ihrem Inneren. Der Hohlraum der Kapillare 1 kann mit dem Hohlraum in dem Zwischenstück 5 verbunden sein. Hierdurch kann der Hohlraum der Kapillare 1 fluidleitend mit dem Hohlraum des Zwischenstücks 5 verbunden sein, so dass ein medizinisches Fluid durch das Zwischenstück 5 in die Kapillare 1 gedrückt werden kann und durch die Öffnung 2 der Kapillare 1 ausgepresst werden kann.

Das Zwischenstück 5 kann mit der Kapillare 1 fest verbunden sein. Das Zwischenstück 5 und die Kapillare 1 können in einer plastisch verformbaren Führung 6 angeordnet und geführt werden. Hierzu können das Zwischenstück 5 und die Kapillare 1 innerhalb der Führung 6 beweglich angeordnet sein. An ihrer distalen Seite weist die Führung 6 eine Auflage 7 in Form einer Scheibe auf. Die Auflage 7 kann an einem Patienten befestigt werden, um die Vorrichtung am Patienten zu fixieren. Die Scheibe der Auflage 7 kann mehrere Durchbrechungen aufweisen, die einen Gasaustausch zwischen einer Haut 60 des Patienten und der Umgebung erlaubt. Die distale Unterseite und auch die proximale Oberseite der Auflage 7 können Vertiefungen aufweisen. In diese Vertiefungen kann ein Schwamm (nicht gezeigt) in Form einer Kreisscheibe (gegebenenfalls mit Durchbrechungen) eingesetzt werden, der mit einer desinfizierenden Lösung getränkt werden kann. Hiermit kann die Oberfläche der Haut 60 und damit die Eintrittsstelle der Kapillare 1 in den Körper des Patienten keimfrei gehalten werden. Der Schwamm kann Teil der Vorrichtung sein.

In der Führung 6 ist ein Fixierungselement 8 zum Fixieren des Zwischenstücks 5 an der Führung 6 angeordnet. Wenn das Zwischenstück 5 mit dem Fixierungselement 8 an der Führung 6 fixiert ist, kann das Zwischenstück 5 nicht mehr gegen die und in der Führung 6 bewegt werden und kann die Kapillare 1 folglich nicht mehr gegen die Führung 6 axial verschoben werden.

Im Inneren der Kapillare 1 und des Zwischenstücks 5 kann ein herausziehbarer Kern 9 angeordnet sein, der die Öffnung 2 der Kapillare 1 verschließt. Der Kern 9 kann einen geringeren Innendurchmesser aufweisen als die Kapillare 1. Die Öffnung 2 ist vorzugsweise zumindest zu 50 % verschlossen. Der Kern 9 kann hierzu durch die Öffnung 2 ragen, um eine Bewegung des Kerns 9 gegen die Öffnung 2 beim Biegen der Kapillare 1 auszugleichen. Es kann hierzu ausreichen, wenn der Kern 9 maximal 3 mm aus der Öffnung 2 der geraden Kapillare 1 hervorsteht, bevorzugt, wenn der Kern 9 maximal 1 mm aus der Öffnung 2 der geraden Kapillare 1 hervorsteht. Der Kern 9 kann aus dem proximalen Ende des Zwischenstücks 5 herausgezogen werden, um die Öffnung 2 der Kapillare 1 zu öffnen.

Das Fixierungselement 8 kann eine Außengewinde aufweisen und nach Art einer Schraube in einen passenden Sitz 10 mit einem Innengewinde eingeschraubt werden, um das Zwischenstück 5 in dem Sitz 10 und damit an der Führung 6 zu fixieren. Hierzu kann das Zwischenstück 5 durch den Sitz 10 geführt sein. Die Führung 6 kann eine Aufnahme 12 aufweisen, in der der Sitz 10 eingesteckt oder befestigt ist.

Am Übergang von dem Zwischenstück 5 zu der Kapillare 1 kann ein hohler Konus 13 angeordnet sein. Der hohle Konus 13 kann den Innendurchmesser der Hohlräume der Kapillare 1 und des Zwischenstücks 5 ineinander überführen. Hierzu kann sich der Innenraum des hohlen Konus 13 in Richtung des Hohlraums der Kapillare 1 konisch verjüngen.

An einer proximalen Seite der Führung 6 kann ein Anschlag 14 in Form einer abstehenden Scheibe angeordnet sein, die eine Handhabung der Vorrichtung vereinfacht.

Der Kern 9 kann an seinem proximalen Ende mit einer Befestigungskappe 16 verbunden sein, die auf einen Adapter 18 mit einem passenden Außengewinde 20 geschraubt werden kann. Hierzu kann in der Befestigungskappe 16 ein zum Außengewinde 20 des Adapters 18 passendes Innengewinde 42 angeordnet sein. Der Adapter 18 kann am proximalen Ende des Zwischenstücks 5 befestigt sein. Über die Befestigungskappe 16 und den Adapter 18 ist der Kern 9 mit dem Zwischenstück 5 und damit mit der Kapillare 1 verbunden. Durch Abschrauben der Befestigungskappe 16 von dem Adapter 18 kann der Kern 9 von dem Zwischenstück 5 und der Kapillare 1 gelöste werden. Nach dem Lösen der Befestigungskappe 16 kann der Kern 9 aus dem Zwischenstück 5 und der Kapillare 1 herausgezogen werden. Dadurch können die Hohlräume im Inneren der Kapillare 1 und des Zwischenstücks 5 freigelegt und die Öffnung 2 geöffnet werden. Hierdurch kann verhindert werden, dass die Öffnung 2 durch eine Verschmutzung zugesetzt oder verstopft wird, bevor der Kern 9 entfernt wurde.

Das Fixierungsmittel 8 kann über einen Drehgriff 22 bedient beziehungsweise aufgeschraubt und gelöst werden. Nach dem Lösen des Fixierungsmittels 8 mit dem Drehgriff 22 kann das Zwischenstück 5 gegen die Führung 6 bewegt werden.

Die distale Unterseite der Auflage 6 bildet eine Auflagefläche 24 zur Auflage auf der Haut 60 beziehungsweise der Oberfläche eines Patienten.

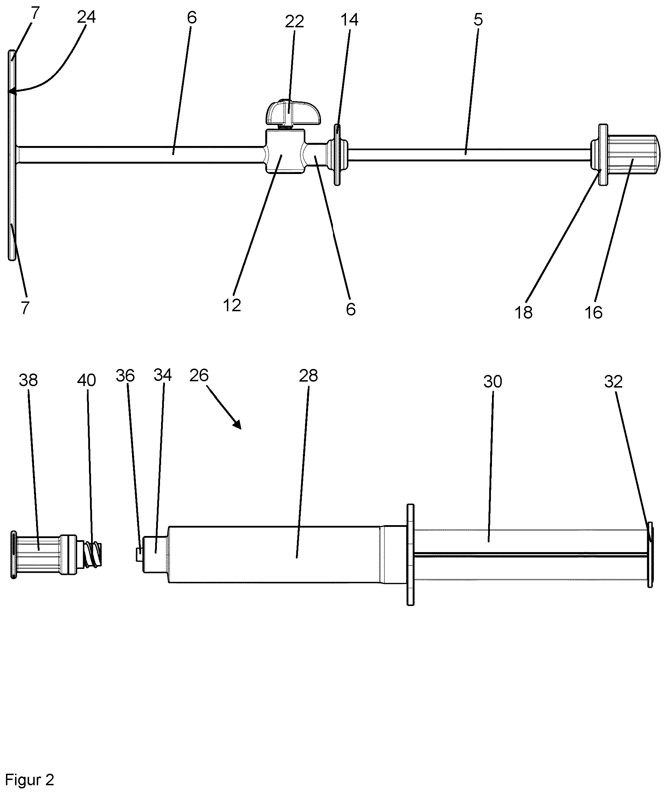

Eine Spritze 26 (In Figur 2 unten rechts) kann Teil der Vorrichtung aber auch ein separates Teil sein, mit der ein pharmazeutisches Fluid in die Vorrichtung eingespritzt werden kann. Die Spritze 26 kann einen Behälter 28 mit einem zylindrischen Innenraum für das pharmazeutische Fluid aufweisen. In dem zylindrischen Innenraum des Behälters 28 kann ein Kolben 30 axial beweglich angeordnet sein, der über einen Griffstempel 32 in den Behälter 28 gedrückt werden kann, um ein medizinisches beziehungsweise pharmazeutisches Fluid aus dem Behälter 28 auszupressen. An der Vorderseite der Spritze 26 kann ein Stutzen 34 mit einem Innengewinde angeordnet sein, um die Spritze 26 mit dem Adapter 18 verbinden zu können. An der Spitze des Stutzens 34 kann ein Verschluss 36 zum Verschließen des Innenraums des Behälters 28 an dessen Vorderseite angeordnet sein.

Zum Verbinden des Stutzens 34 mit dem Adapter 18 des Zwischenstücks 5 kann ein Anschlussadapter 38 mit einem Außengewinde 40 vorgesehen sein. Das Außengewinde 40 des Anschlussadapters 38 kann zum Herstellen einer fluiddichten Verbindung mit dem Innengewinde des Stutzens 34 der Spritze 26 verwendet werden. Über den Anschlussadapter 38 kann so eine fluiddichte Verbindung zwischen dem Innenraum des Behälters 28 der Spritze 26 und dem Hohlraum des Zwischenstücks 5 hergestellt werden.

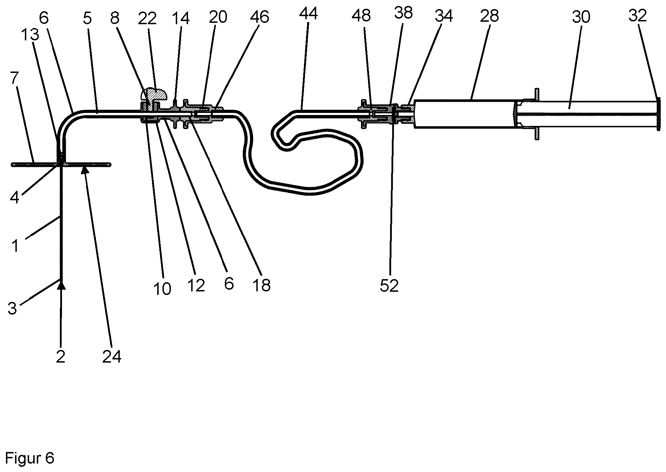

Zur Verlängerung der Verbindung zwischen der Spritze 26 und dem Zwischenstück 5 kann ein Schlauch 44 verwendet werden. Der Schlauch 44 hat zudem den Vorteil, dass er verformbar ist, so dass bei einer Verbindung der Spritze 26 mit dem Zwischenstück 5 über den Schlauch 44 keine Kräfte von der Spritze 26 auf das Zwischenstück 5 übertragen werden, solange der Schlauch lose und nicht gespannt ist. Der Schlauch 44 kann einen Gegenadapter 46 zur Verbindung mit dem Adapter 18 am Zwischenstück 5 aufweisen. Ferner kann der Schlauch 44 einen Anschluss 48 zum Verbinden mit dem Anschlussadapter 38 aufweisen. Hierdurch kann der Schlauch 44 eine fluiddichte Verbindung zwischen der Spritze 26 und dem Zwischenstück bereitstellen (siehe Figuren 6, 8, 9 und 10).

Im Inneren des Anschlussadapters 38 kann ein Filter 52 angeordnet sein, um einen Durchtritt von Partikeln und/oder Keimen in das Zwischenstück 5 zu verhindern. Bevorzugt ist dieser Filter 52 ein Sterilfilter. In dem Adapter 18 oder im distalen Ende des Hohlraums des Zwischenstücks 5 kann zum gleichen Zweck ein Sterilfilter (nicht gezeigt) angeordnet sein.

Der Ausgangszustand der ersten erfindungsgemäßen Vorrichtung ist in den Figuren 1 und 2 gezeigt. Der Kern 9 kann den Hohlraum der Kapillare 1 und den Hohlraum des Zwischenstücks 5 verschließen. Der Kern 9 kann über die Befestigungskappe 16 mit dem Adapter 18 am Zwischenstück 5 verbunden sein. Die Kapillare 1 kann vollständig in die Führung 6 zurückgezogen sein. Das Zwischenstück 5 kann proximal aus der Führung 6 herausragen. Die Führung 6 kann, wie in Figur 1 gezeigt, zur Anwendung der Vorrichtung gebogen oder gekrümmt werden. Dabei wird auch die Kapillare 1 und der Kern 9 darin gebogen oder gekrümmt. Die Kapillare 1 und der Kern 9 sowie das Zwischenstück 5 sollen zerstörungsfrei zu biegen oder zu krümmen sein.

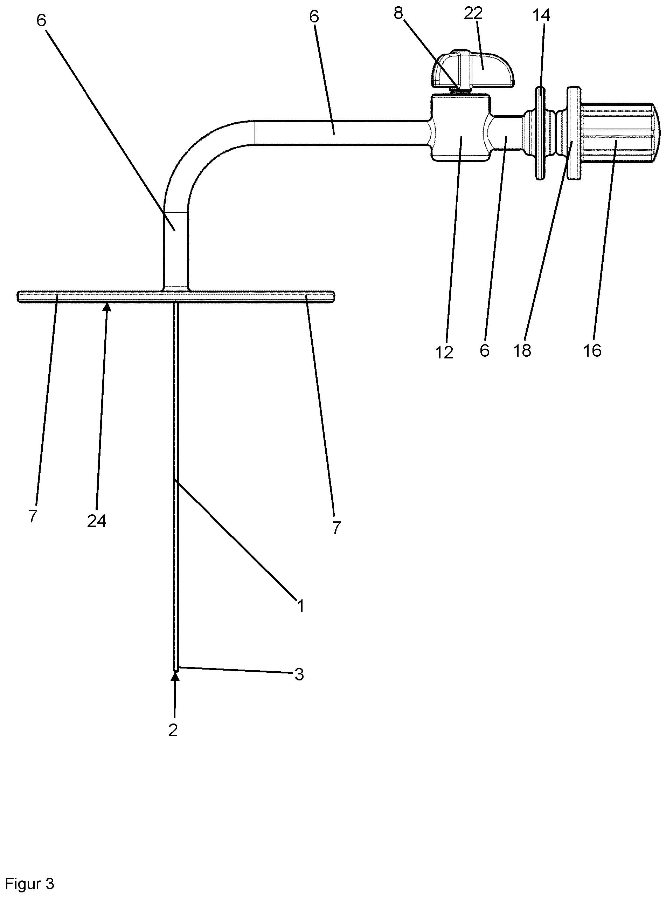

Als nächstes kann das Fixierungselement 8 gelöst werden. Danach kann das Zwischenstück 5 in die Führung 6 eingeschoben werden. Die Kapillare 1 kann dabei aus der distalen Vorderseite der Führung 6 austreten. Beim Einschieben des Zwischenstücks 5 kann das Zwischenstück 5 von der Führung 6 gekrümmt werden. Wenn die Länge der aus der Führung 6 ragenden Kapillare 1 so lange ist, wie der Anwender das wünscht, kann das Zwischenstück 5 wieder mit dem Fixierungselement 8 an der Führung 6 befestigt werden. Danach kann das Zwischenstück 5 nicht mehr gegen die Führung 6 bewegt werden und die Länge der aus der Führung 6 vorstehenden Kapillare 1 ist festgelegt. In Figur 3 ist das Zwischenstück 5 maximal in die Führung 6 eingeschoben. Dann stößt der Adapter 18 an den Anschlag 14 und blockiert ein weiteres Einschieben, bevor der hohle Konus 13 in dem distalen Ende der Führung 6 verkeilt. Diese Situation ist in Figur 3 gezeigt.

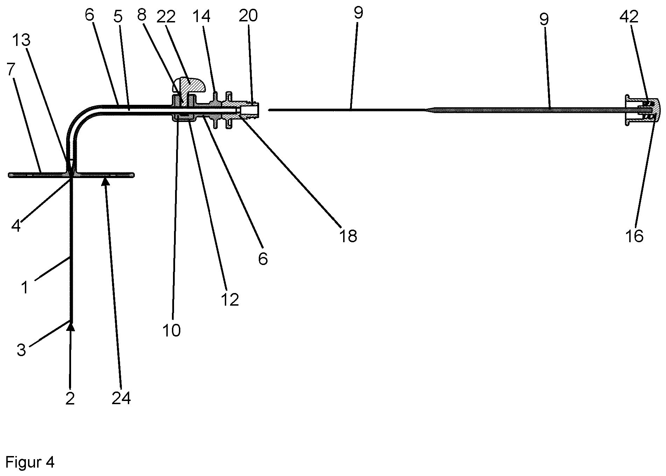

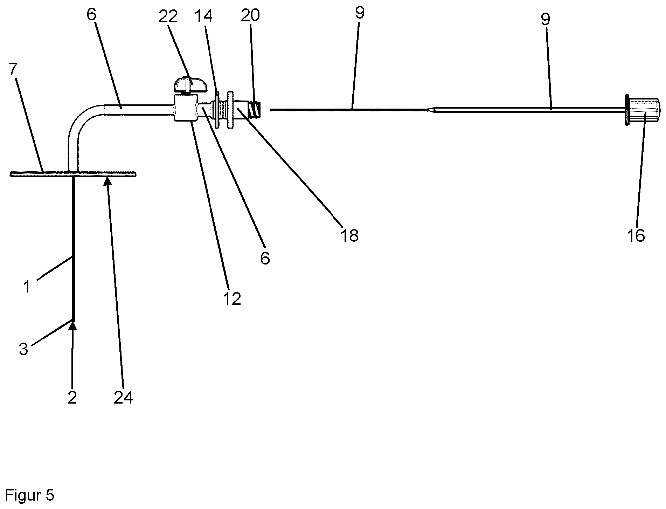

Als nächstes kann die Befestigungskappe 16 vom Adapter 18 abgeschraubt werden und der Kern 9 aus der Kapillare 1 und dem Zwischenstück 5 herausgezogen werden. Diese Situation in den Figuren 4 und 5 gezeigt. Die Öffnung 2 am distalen Ende 3 der Kapillare 1 ist dann geöffnet.

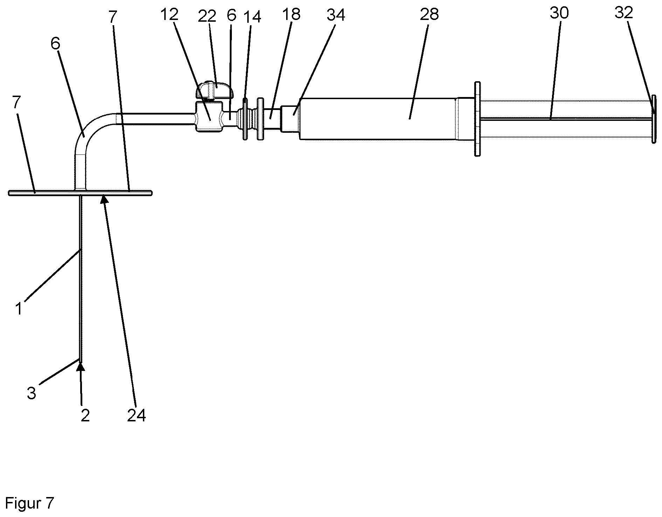

Nun kann die Spritze 26, die mit einem pharmazeutischen Fluid gefüllt sein kann, direkt (siehe Figur 7) oder über den Schlauch 44 (siehe Figuren 6 und 8 bis 10) mit dem Zwischenstück 5 verbunden werden. Diese Situation ist in den Figuren 6 und 7 dargestellt.

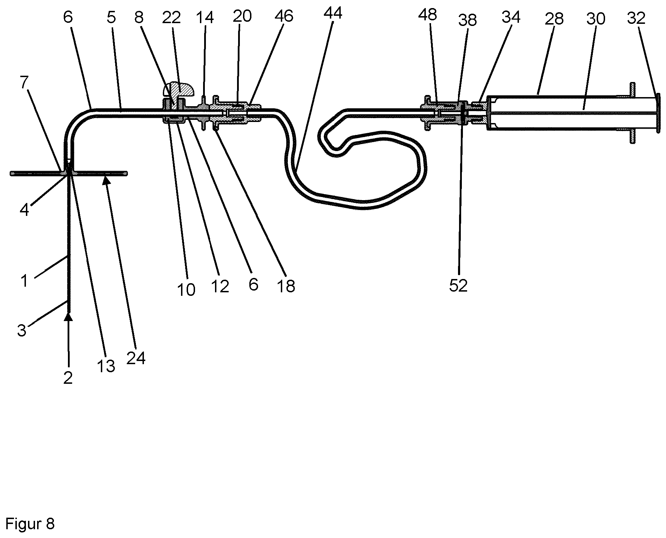

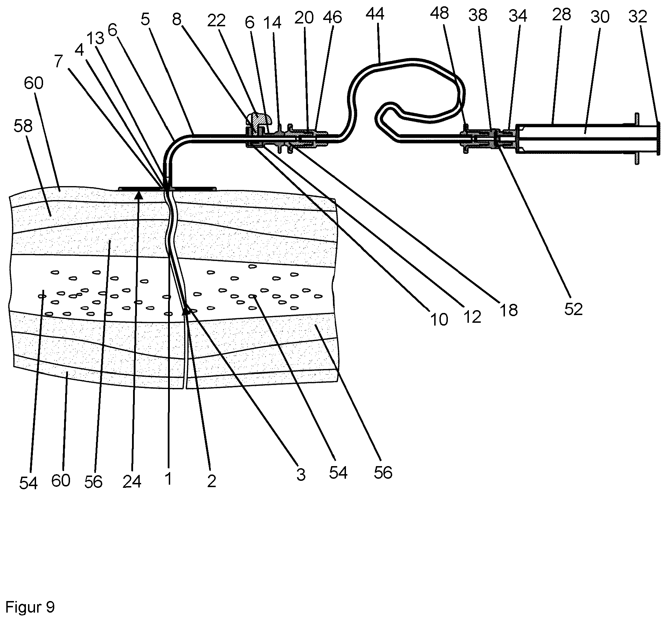

Das Innere des Behälters 28 der Spritze 26 kann nun über den Adapter 18 und gegebenenfalls den Schlauch 44 in das Zwischenstück 5 und die Kapillare 1 eingespritzt werden. Dazu kann der Kolben 30 in den Behälter 28 eingedrückt werden. Diese Situation ist in den Figuren 8 und 9 gezeigt. Alternativ zu der Spritze 26 kann auch ein Tropf (nicht gezeigt) an den Adapter 18 angeschlossen werden, um ein medizinisches Fluid in die Vorrichtung einzuspeisen.

Zuvor kann die Kapillare 1 in einen Spalt einer gerichteten Fraktur oder in eine Bohrung eingesetzt werden. Dabei kann die Kapillare 1 durch eine plastische Verformung aber auch durch eine elastische Verformung an die anatomischen Gegebenheiten angepasst werden. Die Vorrichtung kann dabei auf der Oberfläche des Patienten fixiert werden, indem die Auflage 7 mit Klebstreifen oder Pflastern (nicht gezeigt) auf der Haut 60 des Patienten fixiert wird. Alternativ kann die Auflage auch auf die Haut 60 des Patienten genäht werden. Die Kapillare 1 kann durch die Haut 60, durch das Weichgewebe 58 und durch den Knochen 56 bis in den Markraum 54 des zu behandelnden Patienten reichen. Die Öffnung 2 der Kapillare 1 kann im Markraum 54 angeordnet sein. Diese Situation ist in den Figuren 9, 10 und 14 gezeigt.

Durch die Kapillare 1 kann so das pharmazeutische Fluid durch die Öffnung 2 in den Markraum 54 geleitet werden und dort zur medizinischen Behandlung eingesetzt werden. Nach Abschluss der Behandlung kann die Kapillare 1 wieder aus dem Patienten herausgezogen werden und die Wundöffnung verheilen. Zur Vermeidung von Keimeintritten während der Behandlung kann an der distalen Seite der Auflage 7 ein Schwamm oder ein Vlies (nicht gezeigt) enthaltend ein Desinfektionsmittel oder ein Antiseptikum angeordnet sein.

Die Kapillare 1 kann aus einem Edelstahl oder Kunststoff bestehen. Das Zwischenstück 5 kann auch Edelstahl oder Kunststoff bestehen. Die Führung 6 besteht vorzugsweise aus Kunststoff. In dem Kunststoff kann eine Metallarmierung zur besseren plastischen Verformbarkeit der Führung 6 enthalten sein. Die restlichen Teile, wie die Spritze 26 und der Schlauch 44, können aus Kunststoff gefertigt werden.

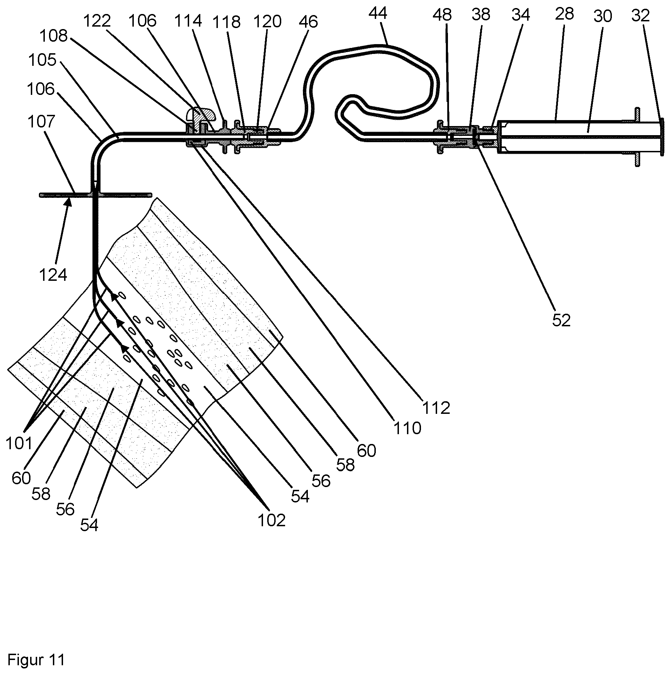

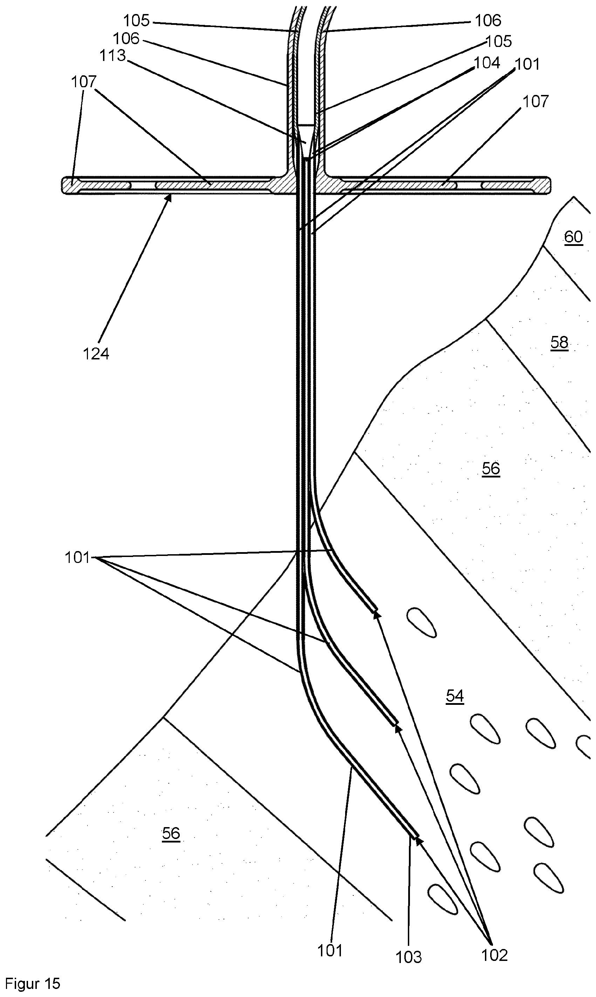

Die Figuren 11 und 15 zeigen schematische Querschnittansichten einer zweiten erfindungsgemäßen Vorrichtung, die im Gegensatz zur ersten erfindungsgemäßen Vorrichtung nicht nur eine sondern drei Kapillaren 101 zur Applikation eines medizinischen Fluids aufweist. Jeder der drei Kapillaren 101 kann eine Öffnung 102 im distalen Ende 103 der Kapillaren 101 aufweisen. Es können aber auch mehrere Öffnungen (nicht gezeigt) in den Wandungen der Kapillaren 101 vorgesehen sein.

Die zweite erfindungsgemäße Vorrichtung ist dabei im bei einem Patienten eingesetzten Zustand gezeigt. Dabei wird jedoch lediglich die Lage symbolisch in dem Körper des Patienten gezeigt. Die Vorrichtung würde im Normalfall also nicht an einer abgetrennten Extremität angewendet, so wie man das aus den Figuren 11 und 15 schließen könnte. Die Einbausituation entspricht vielmehr der nach den Figuren 9, 10 und 14. Die zweite erfindungsgemäße Vorrichtung wird also vorzugsweise derart angewendet, dass die Öffnungen 103 der Kapillaren 101 an unterschiedlichen Stellen im Markraum 54 des Knochens 56 und/oder im Knochen 56 angeordnet werden und in den Markraum 54 beziehungsweise den Knochen 56 einmünden.

Mit der zweiten erfindungsgemäßen Vorrichtung kann das pharmazeutische Fluid auf diese Weise in verschiedenen Tiefen oder an verschiedenen Stellen im Markraum 54 und/oder Knochen 56 des Patienten abgegeben werden.

An den distalen Enden 103 gegenüberliegenden proximalen Enden 104 der Kapillaren 101 können die Kapillaren 101 mit einem hohlen Zwischenstück 105 verbunden sein. Jede der Kapillaren 101 begrenzt einen Hohlraum in ihrem Inneren. Die Hohlräume der Kapillaren 101 können mit dem Hohlraum in dem Zwischenstück 105 verbunden sein. Hierdurch können die Hohlräume der Kapillaren 101 fluidleitend mit dem Hohlraum des Zwischenstücks 105 verbunden sein, so dass ein medizinisches Fluid durch das Zwischenstück 105 in die Kapillaren 101 gedrückt werden kann und durch die Öffnungen 102 der Kapillaren 101 an unterschiedlichen Stellen ausgepresst werden kann.

Bevorzugt können die Kapillaren 101 bereichsweise als Bündel zusammengefasst sein. Hierzu können die Kapillaren 101 ausgehend von dem Zwischenstück 105 miteinander verbunden sein. Die Verbindung kann sich bis zu den distalen Enden 103 der Kapillaren 101 erstrecken. Bevorzugt erstreckt sich die Verbindung jedoch nur bis zu etwa der Hälfte der Länge der Kapillaren 101.

Das Zwischenstück 105 kann mit den Kapillaren 101 fest verbunden sein. Das Zwischenstück 105 und die Kapillaren 101 können in einer plastisch verformbaren Führung 106 angeordnet und geführt werden. Hierzu können das Zwischenstück 105 und die Kapillaren 101 innerhalb der Führung 106 beweglich angeordnet sein. An ihrer distalen Seite weist die Führung 106 eine Auflage 107 in Form einer Scheibe auf. Die Auflage 107 kann an einem Patienten befestigt werden, um die Vorrichtung am Patienten zu fixieren. Die Scheibe der Auflage 107 kann mehrere Durchbrechungen aufweisen, die einen Gasaustausch zwischen einer Haut 60 des Patienten und der Umgebung erlaubt. Die Auflage 107 wird also in der Anwendung auf die Haut 60 aufgelegt und nicht, wie in den Figuren 11 und 15 lediglich schematisch dargestellt freischwebend gehalten. Die distale Unterseite und auch die proximale Oberseite der Auflage 107 können Vertiefungen aufweisen. In diese Vertiefungen kann ein Schwamm (nicht gezeigt) in Form einer Kreisscheibe (gegebenenfalls mit Durchbrechungen) eingesetzt werden, der mit einer desinfizierenden Lösung getränkt werden kann. Hiermit kann die Oberfläche der Haut 60 und damit die Eintrittsstelle der Kapillaren 101 in den Körper des Patienten keimfrei gehalten werden. Der Schwamm kann Teil der Vorrichtung sein.

In der Führung 106 ist ein Fixierungselement 108 zum Fixieren des Zwischenstücks 105 an der Führung 106 angeordnet. Wenn das Zwischenstück 105 mit dem Fixierungselement 108 an der Führung 106 fixiert ist, kann das Zwischenstück 105 nicht mehr gegen die und in der Führung 106 bewegt werden und können die Kapillaren 101 folglich nicht mehr axial gegen die Führung 106 verschoben werden.

Im Inneren der Kapillaren 101 und des Zwischenstücks 105 kann im Ausgangszustand (in den Figuren 11 und 15 nicht gezeigt) ein herausziehbarer Kern (nicht gezeigt aber analog dem ersten Ausführungsbeispiel) angeordnet sein, der die Öffnungen 102 der Kapillaren 101 verschließt. Der Kern kann im Unterschied zu dem Kern 9 nach dem ersten Ausführungsbeispiel mehrgliedrig in Äste aufgefächert sein, wobei die in den Kapillaren 101 angeordneten Äste einen geringeren Innendurchmesser aufweisen als die Kapillaren 101. Die Öffnungen 102 sind vorzugsweise zumindest zu 50 % verschlossen. Der Kern beziehungsweise die Äste des Kerns können hierzu durch die Öffnungen 102 aus den Kapillaren 101 ragen, um eine Bewegung des Kerns gegen die Öffnungen 102 beim Biegen der Kapillaren 101 auszugleichen. Es kann hierzu ausreichen, wenn der Kern maximal 3 mm aus den Öffnungen 102 der geraden Kapillaren 101 hervorsteht, bevorzugt, wenn der Kern maximal 1 mm aus den Öffnungen 102 der geraden Kapillaren 101 hervorsteht. Der Kern kann aus dem proximalen Ende des Zwischenstücks 105 herausgezogen werden, um die Öffnungen 102 der Kapillaren 101 zu öffnen.

Das Fixierungselement 108 kann eine Außengewinde aufweisen und nach Art einer Schraube in einen passenden Sitz 110 mit einem Innengewinde eingeschraubt werden, um das Zwischenstück 105 in dem Sitz 110 und damit an der Führung 106 zu fixieren. Hierzu kann das Zwischenstück 105 durch den Sitz 110 geführt sein. Die Führung 106 kann eine Aufnahme 112 aufweisen, in der der Sitz 110 eingesteckt oder befestigt ist.

Am Übergang von dem Zwischenstück 105 zu den Kapillaren 101 kann ein hohler Konus 113 angeordnet sein. Der hohle Konus 113 kann die Hohlräume der Kapillaren 101 und des Zwischenstücks 105 miteinander verbinden und ineinander überführen. Hierzu kann sich der Innenraum des hohlen Konus 113 in Richtung des Hohlraums der Kapillare 101 konisch verjüngen. Die Kapillaren 101 können über die konische Wandung des hohlen Konus 113 mit dem Zwischenstück 105 verbunden sein.

An einer proximalen Seite der Führung 106 kann ein Anschlag 114 in Form einer abstehenden Scheibe angeordnet sein, die eine Handhabung der Vorrichtung vereinfacht.

Der Kern kann im Ausgangszustand (in den Figuren 11 und 15 nicht gezeigt) an seinem proximalen Ende mit einer Befestigungskappe (nicht gezeigt) verbunden sein, die auf einen Adapter 118 mit einem passenden Außengewinde 120 geschraubt werden kann. Hierzu kann in der Befestigungskappe ein zum Außengewinde 120 des Adapters 118 passendes Innengewinde angeordnet sein. Der Adapter 118 kann am proximalen Ende des Zwischenstücks 105 befestigt sein. Über die Befestigungskappe und den Adapter 118 ist der Kern mit dem Zwischenstück 105 und damit mit den Kapillaren 101 verbunden. Durch Abschrauben der Befestigungskappe von dem Adapter 118 kann der Kern von dem Zwischenstück 105 und den Kapillaren 101 gelöste werden. Nach dem Lösen der Befestigungskappe kann der Kern analog dem ersten Ausführungsbeispiel aus dem Zwischenstück 105 und den Kapillaren 101 herausgezogen werden. Dadurch können die Hohlräume im Inneren der Kapillaren 101 und des Zwischenstücks 105 freigelegt und die Öffnungen 102 geöffnet werden. Hierdurch kann verhindert werden, dass die Öffnungen 102 durch Verschmutzung zugesetzt oder verstopft wird, bevor der Kern entfernt wurde.

Das Fixierungsmittel 108 kann über einen Drehgriff 122 bedient beziehungsweise aufgeschraubt und gelöst werden. Nach dem Lösen des Fixierungsmittels 108 mit dem Drehgriff 122 kann das Zwischenstück 105 gegen die Führung 106 bewegt werden.

Die distale Unterseite der Auflage 106 bildet eine Auflagefläche 124 zur Auflage auf der Haut 60 beziehungsweise der Oberfläche eines Patienten.

Eine Spritze 26 (In Figur 11 und 15 rechts) kann Teil der Vorrichtung aber auch ein separates Teil sein, mit der ein pharmazeutisches Fluid in die Vorrichtung eingespritzt werden kann. Die Spritze 26 kann einen Behälter 28 mit einem zylindrischen Innenraum für das pharmazeutische Fluid aufweisen. In dem zylindrischen Innenraum des Behälters 28 kann ein Kolben 30 axial beweglich angeordnet sein, der über einen Griffstempel 32 in den Behälter 28 gedrückt werden kann, um ein medizinisches beziehungsweise pharmazeutisches Fluid aus dem Behälter 28 auszupressen. An der Vorderseite der Spritze 26 kann ein Stutzen 34 mit einem Innengewinde angeordnet sein, um die Spritze 26 mit dem Adapter 118 verbinden zu können. An der Spitze des Stutzens 34 kann ein Verschluss 36 zum Verschließen des Innenraums des Behälters 28 an dessen Vorderseite angeordnet sein.

Zum Verbinden des Stutzens 34 mit dem Adapter 118 des Zwischenstücks 105 kann ein Anschlussadapter 38 mit einem Außengewinde 40 vorgesehen sein. Das Außengewinde 40 des Anschlussadapters 38 kann zum Herstellen einer fluiddichten Verbindung mit dem Innengewinde des Stutzens 34 der Spritze 26 verwendet werden. Über den Anschlussadapter 38 kann so eine fluiddichte Verbindung zwischen dem Innenraum des Behälters 28 der Spritze 26 und dem Hohlraum des Zwischenstücks 105 hergestellt werden.

Zur Verlängerung der Verbindung zwischen der Spritze 26 und dem Zwischenstück 105 kann ein Schlauch 44 verwendet werden. Der Schlauch 44 hat zudem den Vorteil, dass er verformbar ist, so dass bei einer Verbindung der Spritze 26 mit dem Zwischenstück 105 über den Schlauch 44 keine Kräfte von der Spritze 26 auf das Zwischenstück 105 übertragen werden, solange der Schlauch lose und nicht gespannt ist. Der Schlauch 44 kann einen Gegenadapter 46 zur Verbindung mit dem Adapter 118 am Zwischenstück 105 aufweisen. Ferner kann der Schlauch 44 einen Anschluss 48 zum Verbinden mit dem Anschlussadapter 38 aufweisen. Hierdurch kann der Schlauch 44 eine fluiddichte Verbindung zwischen der Spritze 26 und dem Zwischenstück bereitstellen.

Im Inneren des Anschlussadapters 38 kann ein Filter 52 angeordnet sein, um einen Durchtritt von Partikeln und/oder Keimen in das Zwischenstück 105 zu verhindern. Bevorzugt ist dieser Filter 52 ein Sterilfilter. In dem Adapter 118 oder im distalen Ende des Hohlraums des Zwischenstücks 105 kann zum gleichen Zweck ein Sterilfilter (nicht gezeigt) angeordnet sein.

Der Ablauf eines erfindungsgemäßen Verfahrens gleicht bei dem zweiten Ausführungsbeispiel grundsätzlich dem des ersten Ausführungsbeispiels. Beim Ziehen des Kerns aus dem Zwischenstück 105 und den Kapillaren 101 werden beim zweiten Ausführungsbeispiel nicht nur eine sondern alle Öffnungen 102 der Kapillaren 101 geöffnet.

Zuvor können die Kapillaren 101 in einen Spalt einer gerichteten Fraktur oder in eine Bohrung eingesetzt werden, wobei die distalen Enden 103 der Kapillaren 101 und damit die Öffnungen 102 an unterschiedlichen Stellen platziert werden. Dabei können die Kapillaren 101 durch eine plastische Verformung aber auch durch eine elastische Verformung an die anatomischen Gegebenheiten angepasst werden und in die gewünschten unterschiedlichen Stellen erreichen. Die Vorrichtung kann dabei auf der Oberfläche des Patienten fixiert werden, indem die Auflage 107 mit Klebstreifen oder Pflastern (nicht gezeigt) auf der Haut 60 des Patienten fixiert wird. Alternativ kann die Auflage auch auf die Haut 60 des Patienten genäht werden. Die Kapillaren 101 können durch die Haut 60, durch das Weichgewebe 58 und durch den Knochen 56 bis in den Markraum 54 des zu behandelnden Patienten reichen. Die Öffnungen 102 der Kapillaren 101 können so an allen gewünschten Stellen im Markraum 54 oder auch im Knochen 56 angeordnet werden. Diese Situation ist in den Figuren 11 und 15 gezeigt.

Durch die Kapillaren 101 kann so das pharmazeutische Fluid durch die Öffnungen 102 in den Markraum 54 geleitet werden und dort zur medizinischen Behandlung eingesetzt werden. Nach Abschluss der Behandlung können die Kapillaren 101 wieder aus dem Patienten herausgezogen werden und die Wundöffnung verheilen. Zur Vermeidung von Keimeintritten während der Behandlung kann an der distalen Seite der Auflage 107 ein Schwamm oder ein Vlies (nicht gezeigt) enthaltend ein Desinfektionsmittel oder ein Antiseptikum angeordnet sein.

Die Kapillaren 101 können aus einem Edelstahl oder Kunststoff bestehen. Das Zwischenstück 105 kann auch Edelstahl oder Kunststoff bestehen. Die Führung 106 besteht vorzugsweise aus Kunststoff. In dem Kunststoff kann eine Metallarmierung zur besseren plastischen Verformbarkeit der Führung 106 enthalten sein. Die restlichen Teile, wie die Spritze 26 und der Schlauch 44, können aus Kunststoff gefertigt werden.

Als pharmazeutisches Fluid kann bei allen Ausführungen vorzugsweise eine Lösung enthaltend zumindest ein Antibiotikum oder ein Antimykotikum oder eine Mischung verschiedener Antibiotika und/oder Antimykotika verwendet werden.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 102: Kapillare
- 2, 102: Öffnung
- 3, 103: Distales Ende
- 4, 104: Proximales Ende
- 5, 105: Zwischenstück
- 6, 106: Führung
- 7, 107: Auflage
- 8, 108: Fixierungselement
- 9: Kern
- 10, 110: Sitz
- 12, 112: Aufnahme
- 13, 113: Hohler Konus
- 14, 114: Anschlag
- 16: Befestigungskappe
- 18, 118: Adapter
- 20, 120: Außengewinde
- 22, 122: Drehgriff
- 24, 124: Auflagefläche
- 26: Spritze
- 28: Behälter
- 30: Kolben
- 32: Griffstempel
- 34: Stutzen
- 36: Verschluss
- 38: Anschlussadapter
- 40: Außengewinde
- 42: Innengewinde
- 44: Schlauch
- 46: Gegenadapter
- 48: Anschluss
- 52: Filter
- 54: Markraum
- 56: Knochengewebe
- 58: Weichgewebe
- 60: Haut

## Patentansprüche

1. Vorrichtung zur lokalen Applikation von pharmazeutischen Fluiden, die Vorrichtung aufweisend
mindestens eine Kapillare (1, 101) mit mindestens einer Öffnung (2, 102) im Bereich des distalen Endes (3, 103) der mindestens einen Kapillare (1, 101),
ein hohles Zwischenstück (5, 105), das an einer der mindestens einen Öffnung (2, 102) gegenüberliegenden Seite der mindestens einen Kapillare (1, 101) mit der mindestens einen Kapillare (1, 101) flüssigkeitsdurchlässig verbunden ist,
eine Führung (6, 106) zum Führen des Zwischenstücks (5, 105), wobei die Führung (6, 106) eine distale Auflage (7, 107) zur Auflage auf den Patienten aufweist,
ein Fixierungselement (8, 108) zur Fixierung des Zwischenstücks (5, 105) an der Führung (6, 106), wobei mit dem Fixierungselement (8, 108) eine Fixierung des Zwischenstücks (5, 105) in verschiedenen Positionen relativ zur Führung (6, 106) möglich ist, wobei die Fixierung eine Verschiebung des Zwischenstücks (5, 105) gegen die Führung (6, 106) verhindert und wobei das Fixierungselement (8, 108) eine an der Führung (6, 106) angeordnete Haltevorrichtung oder Klemmvorrichtung ist, oder das Fixierungselement (8, 108) eine Schraube oder eine Flügelschraube ist.

2. Vorrichtung nach Anspruch 1, **dadurch gekennzeichnet, dass**
in einem Hohlraum der mindestens einen Kapillare (1, 101) und des Zwischenstücks (5, 105) ein Kern (9) angeordnet ist, wobei der Kern (9) aus dem Hohlraum der mindestens einen Kapillare (1, 101) entfernbar ist, wobei der Kern (9) mindestens 50 % der Querschnittsfläche der mindestens einen Öffnung (2, 102) verschließt, und wobei der Kern (9) mit dem Zwischenstück (5, 105) derart lösbar verbunden ist, dass die Verbindung eine axiale Bewegung des Kerns (9) im Hohlraum der mindestens einen Kapillare (1, 101) einschränkt oder verhindert.

3. Vorrichtung nach Anspruch 2, **dadurch gekennzeichnet, dass**
der Kern (9) durch einen Adapter (18, 118) aus dem Hohlraum der mindestens einen Kapillare (1, 101) ziehbar ist und bevorzugt auch aus dem Hohlraum des hohlen Zwischenstücks (5, 105) ziehbar ist.

4. Vorrichtung nach einem der vorangehenden Ansprüche, **dadurch gekennzeichnet, dass**
die Vorrichtung einen Schlauch (44) aufweist, der mit dem Zwischenstück (5, 105) flüssigkeitsdurchlässig verbunden oder verbindbar ist, wobei der Schlauch (44) verformbar ist, und/oder
das Zwischenstück (5, 105), insbesondere über den Schlauch (44), mit einem Fluidreservoir flüssigkeitsdurchlässig verbunden oder verbindbar ist, wobei ein pharmazeutisches Fluid aus dem Fluidreservoir unter Druck durch das Zwischenstück (5, 105) und durch die mindestens eine Kapillare (1, 101), und sofern vorhanden auch durch den Schlauch (44), aus der mindestens einen Öffnung (2, 102) der mindestens einen Kapillare (1, 101) pressbar ist.

5. Vorrichtung nach einem der vorangehenden Ansprüche, **dadurch gekennzeichnet, dass**
das Zwischenstück (5, 105) hohlzylinderförmig oder schlauchförmig ist, wobei vorzugsweise das Zwischenstück (5, 105) an seiner mit der mindestens einen Kapillare (1, 101) verbundenen distalen Seite eine Verjüngung aufweist, bevorzugt eine konische Verjüngung, wobei besonders bevorzugt die konische Verjüngung ein hohler Konus (13, 113) ist, und/oder
das Zwischenstück (5, 105) in der Führung (6, 106) axial beweglich angeordnet ist, wenn die Fixierung des Fixierungselements (8, 108) gelöst ist.

6. Vorrichtung nach einem der vorangehenden Ansprüche, **dadurch gekennzeichnet, dass**
der Kern (9) mit einem Gewinde (42) oder einem Bajonettverschluss mit dem Zwischenstück (5, 105) lösbar verbunden ist.

7. Vorrichtung nach einem der vorangehenden Ansprüche, **dadurch gekennzeichnet, dass**
das Fixierungselement (8, 108) eine Klemmvorrichtung mit zusammenschraubbaren Klemmbacken ist, oder
das Fixierungselement (8, 108) eine Schraube ist, wobei die Schraube in einem durch eine Wandung der Führung (6, 106) reichende Bohrung mit einem Innengewinde eingeschraubt oder einschraubbar ist, so dass mit einem vorderen Ende der Schraube das Zwischenstück (5, 105) in der Führung (6, 106) einklemmbar ist.

8. Vorrichtung nach einem der vorangehenden Ansprüche, **dadurch gekennzeichnet, dass**
das Zwischenstück (5, 105) für Flüssigkeiten durchlässig ist, wenn das Zwischenstück (5, 105) mit dem Fixierungselement (8, 108) in der Führung (6, 106) fixiert ist.

9. Vorrichtung nach einem der vorangehenden Ansprüche, **dadurch gekennzeichnet, dass**
der Hohlraum der Führung (6, 106) mindesten so lang ist, wie die Länge der mindestens einen Kapillare (1, 101) beziehungsweise wie die Länge der längsten Kapillare (1, 101) der mindestens einen Kapillare (1, 101), und/oder
der Hohlraum in der Führung (6, 106) am distalen Ende zulaufend ist, insbesondere stetig zulaufend oder konisch zulaufend ist, und dass eine Durchbrechung am distalen Ende des zulaufenden Bereichs, insbesondere an einer Spitze eines Konus angeordnet ist, durch den die mindestens eine Kapillare (1, 101) durch das distale Ende der Führung (6, 106) hindurchgeführt ist oder hindurchführbar ist, wobei bevorzugt durch Durchmesser der Durchbrechung kleiner ist als der Außendurchmesser des Zwischenstücks (5, 105).

10. Vorrichtung nach einem der vorangehenden Ansprüche, **dadurch gekennzeichnet, dass**
die Vorrichtung einen Behälter (28) für das pharmazeutische Fluid aufweist, wobei bevorzugt in dem Behälter (28) ein pharmazeutisches Fluid enthalten ist, besonders bevorzugt enthaltend zumindest einen antibiotischen Wirkstoff, zumindest einen antimykotischen Wirkstoff und/oder zumindest ein Chemotherapeutikum, und/oder
der Kern (9) über wenigstens die am dichtesten am distalen Ende (3, 103) der mindestens einen Kapillare (1, 101) angeordnete Öffnung (2, 102) der mindestens einen Öffnung (2, 102) soweit heraussteht, dass bei einer Biegung der mindestens einen Kapillare (1, 101) diese Öffnung (2, 102) zu mindestens 50 % der Querschnittsfläche dieser Öffnung (2, 102) verschlossen ist.

11. Vorrichtung nach einem der vorangehenden Ansprüche, **dadurch gekennzeichnet, dass**
die mindestens eine Kapillare (1, 101) mindestens zwei Kapillaren (101) sind, wobei die mindestens zwei Kapillaren (101) eine unterschiedliche Länge haben, bevorzugt alle Kapillaren (101) der mindestens zwei Kapillaren (101) eine unterschiedliche Länge haben.

12. Vorrichtung nach einem der vorangehenden Ansprüche, **dadurch gekennzeichnet, dass**
die mindestens eine Kapillare (1, 101) aus Titan, aus Edelstahl oder aus Kunststoff besteht, insbesondere aus einem formstabilen Kunststoff besteht, und/oder
die mindestens eine Kapillare (1, 101) und der Kern (9) verformbar sind, wobei vorzugsweise die mindestens eine Kapillare (1, 101) plastisch verformbar ist.

13. Vorrichtung nach einem der vorangehenden Ansprüche, **dadurch gekennzeichnet, dass**
am distalen Ende der Führung (6, 106) eine poröse schwammartige Scheibe angeordnet ist, wobei vorzugsweise die poröse schwammartige Scheibe eine antiseptische Flüssigkeit, insbesondere eine antiseptische Lösung enthält.

14. Verfahren zur Vorbereitung der Vorrichtung nach einem der vorangehenden Ansprüche vor einer medizinischen Anwendung, wobei das Verfahren nicht zur Behandlung des menschlichen oder eines tierischen Körpers eingesetzt wird, das Verfahren aufweisend die folgenden chronologisch nacheinander ablaufenden Schritten:
A) Herausdrücken der mindestens einen Kapillare (1, 101) aus der Führung (6, 106) durch Einschieben des Zwischenstücks (5, 105) in die Führung (6, 106); und
B) Fixieren des Zwischenstücks (5, 105) in der Führung (6, 106) mit dem Fixierungselement (8, 108).

15. Verfahren nach Anspruch 14, **dadurch gekennzeichnet, dass**
in einem Schritt C) nach Schritt B) oder nach Schritt A) ein Umformen der mindestens einen Kapillare (1, 101) durch eine plastische Verformung der mindestens einen Kapillare (1, 101) erfolgt.

## Claims

1. A device for local administration of pharmaceutical fluids, the device comprising
at least one capillary (1, 101) with at least one opening (2, 102) in the region of the distal end (3, 103) of the at least one capillary (1, 101),
a hollow intermediate piece (5, 105), which is connected with the at least one capillary (1, 101) so as to allow passage of liquid,
a guide (6, 106) for guiding the intermediate piece (5, 105) and the at least one capillary (1, 101), wherein the guide (6, 106) comprising a distal support (7, 107) for resting on the patient,
a fixing element (8, 108) for fixing the intermediate piece (5, 105) to the guide (6, 106), wherein the fixing element (8, 108) allows fixing of the intermediate piece (5, 105) in various positions relative to the guide (6, 106), wherein said fixing prevents displacement of the intermediate piece (5, 105) in relation to the guide (6, 106).

2. The device according to Claim 1, **characterized in that**
a core (9) is arranged in a cavity of the at least one capillary (1, 101) and of the intermediate piece (5, 105), wherein the core (9) is removable from the cavity of the at least one capillary (1, 101), wherein the core (9) closes at least 50 % of the cross-sectional area of the at least one opening (2, 102), and wherein the core (9) is releasably connected with the intermediate piece (5, 105) in such a way that the connection limits or prevents axial movement of the core (9) in the cavity of the at least one capillary (1, 101).

3. The device according to Claim 2, **characterized in that**
the core (9) is capable of being drawn out of the cavity of the at least one capillary (1, 101) by an adapter (18, 118) and preferably also of being drawn out of the cavity of the hollow intermediate piece (5, 105).

4. The device according to any one of the preceding claims, **characterized in that**
the device further comprising a tube (44), which is connected or connectable with the intermediate piece (5, 105) so as to allow passage of liquid, wherein the tube (44) is deformable, and/or
the intermediate piece (5, 105) is connected or connectable for passage of liquid with a fluid reservoir, in particular via the hose (44), wherein a pharmaceutical fluid is capable of being forced out of the fluid reservoir under pressure through the intermediate piece (5, 105) and through the at least one capillary (1, 101) and, where present, also through the tube (44) out of the at least one opening (2, 102) of the at least one capillary (1, 101).

5. The device according to any one of the preceding claims, **characterized in that**
the intermediate piece (5, 105) is hollow-cylindrical or tubular, wherein preferably the intermediate piece (5, 105) has a taper at its distal end connected to the at least one capillary (1, 101), preferably a conical taper, wherein particularly preferably the conical taper is a hollow cone (13, 113), and/or
the intermediate piece (5, 105) is arranged axially movably in the guide (6, 106) when the fixing provided by the fixing element (8, 108) is released.

6. The device according to any one of the preceding claims, **characterized in that**
the core (9) is releasably connected with the intermediate piece (5, 105) by a thread (42) or a bayonet closure.

7. The device according to any one of the preceding claims, **characterized in that**
the fixing element (8, 108) is a clamping device having clamping jaws capable of being screwed together, or
the fixing element (8, 108) is a screw, wherein the screw is screwed or screwable into a bore with inner thread extending through a wall of the guide (6, 106), such that the intermediate piece (5, 105) is capable of being clamped in the guide (6, 106) with a front end of the screw.

8. The device according to any one of the preceding claims, **characterized in that**
the intermediate piece (5, 105) allows the passage of liquids when the intermediate piece (5, 105) is fixed by the fixing element (8, 108) in the guide (6, 106).

9. The device according to any one of the preceding claims, **characterized in that**
the cavity of the guide (6, 106) is at least as long as the length of the at least one capillary (1, 101) or as the length of the longest capillary (1, 101) of the at least one capillary (1, 101), and/or
the cavity in the guide (6, 106) tapers at the distal end, in particular continuously tapers or conically tapers, and **in that** a hole is arranged at the distal end of the tapering region, in particular at a tip of a cone, through which hole the at least one capillary (1, 101) is guided or guidable through the distal end of the guide (6, 106), wherein preferably the diameter of the hole is smaller than the external diameter of the intermediate piece (5, 105).

10. The device according to any one of the preceding claims, **characterized in that**
the device comprises a container (28) for the pharmaceutical fluid, wherein a pharmaceutical fluid particularly preferably containing at least one antibiotic active ingredient, at least one antimycotic active ingredient and/or at least one chemotherapeutic agent, is preferably contained in the container (28), and/or
the core (9) projects beyond an at least one distal opening (2, 102) being arranged closest to the distal end (3, 103) of the at least one capillary (1, 101) of the at least one opening (2, 102) to the extent that, on bending of the at least one capillary (1, 101), this at least one distal opening (2, 102) is closed over at least 50 % of the cross-sectional area of this opening (2, 102).

11. The device according to any one of the preceding claims, **characterized in that**
the at least one capillary (1, 101) is at least two capillaries (101), wherein the at least two capillaries (101) have different lengths, preferably all the capillaries (101) of the at least two capillaries (101) have different lengths.

12. The device according to any one of the preceding claims, **characterized in that**
the at least one capillary (1, 101) consists of titanium, of special steel or of plastics material, in particular of a dimensionally stable plastics material, and/or
the at least one capillary (1, 101) and if present the core (9) are deformable, wherein preferably the at least one capillary (1, 101) is plastically deformable.

13. The device according to any one of the preceding claims, **characterized in that**
a porous sponge-like disk is arranged at the distal end of the guide (6, 106), wherein the porous sponge-like disk preferably contains an antiseptic liquid, in particular an antiseptic solution.

14. A method for preparing the device according to any one of the preceding claims prior to medical use, wherein the method not being used for medical treatment of a human or animal body, the method comprising the following steps taking place in a chronological sequence:
A) pushing the at least one capillary (1, 101) out of the guide (6, 106) by inserting the intermediate piece (5, 105) into the guide (6, 106); and
B) fixing the intermediate piece (5, 105) in the guide (6, 106) with the fixing element (8, 108).

15. The method according to Claim 14, **characterized in that**
in a step C) after step B) or after step A) the at least one capillary (1, 101) is shaped by plastic deformation of the at least one capillary (1, 101).

## Revendications

1. Dispositif pour l'application locale de fluides pharmaceutiques, le dispositif présentant au moins un tube capillaire (1, 101) comportant au moins une ouverture (2, 102) dans la zone de l'extrémité distale (3, 103) de l'au moins un tube capillaire (1, 101),
une pièce intermédiaire (5, 105) creuse qui est reliée de manière perméable aux liquides à l'au moins un tube capillaire (1, 101) sur un côté de l'au moins un tube capillaire (1, 101), lequel côté est opposé à l'au moins une ouverture (2, 102),
un guide (6, 106) pour le guidage de la pièce intermédiaire (5, 105), dans lequel le guide (6, 106) présente un appui distal (7, 107) pour l'appui sur le patient,
un élément de fixation (8, 108) pour la fixation de la pièce intermédiaire (5, 105) sur le guide (6, 106), dans lequel, au moyen de l'élément de fixation (8, 108), une fixation de la pièce intermédiaire (5, 105) dans différentes positions par rapport au guide (6, 106) est possible, dans lequel la fixation empêche un coulissement de la pièce intermédiaire (5, 105) contre le guide (6, 106) et dans lequel l'élément de fixation (8, 108) est un dispositif de maintien ou un dispositif de serrage disposé sur le guide (6, 106), ou l'élément de fixation (8, 108) est une vis ou une vis à oreilles.

2. Dispositif selon la revendication 1, **caractérisé en ce que**
un noyau (9) est disposé dans une cavité de l'au moins un tube capillaire (1, 101) et de la pièce intermédiaire (5, 105), dans lequel le noyau (9) peut être enlevé de la cavité de l'au moins un tube capillaire (1, 101), dans lequel le noyau (9) ferme au moins 50 % de la surface de section transversale de l'au moins une ouverture (2, 102), et dans lequel le noyau (9) est relié de manière amovible à la pièce intermédiaire (5, 105) de telle sorte que la liaison limite ou empêche un mouvement axial du noyau (9) dans la cavité de l'au moins un tube capillaire (1, 101).

3. Dispositif selon la revendication 2, **caractérisé en ce que**
au moyen d'un adaptateur (18, 118), le noyau (9) peut être retiré de la cavité de l'au moins un tube capillaire (1, 101) et peut de préférence également être retiré de la cavité de la pièce intermédiaire (5, 105) creuse.

4. Dispositif selon l'une des revendications précédentes, **caractérisé en ce que**
le dispositif présente un tuyau (44) qui est relié ou peut être relié à la pièce intermédiaire (5, 105) de manière perméable aux liquides, dans lequel le tuyau (44) est déformable, et/ou la pièce intermédiaire (5, 105) est reliée ou peut être reliée de manière perméable aux liquides à un réservoir pour fluide, en particulier par l'intermédiaire du tuyau (44), dans lequel un fluide pharmaceutique peut être comprimé sous pression à partir du réservoir pour fluide, à travers la pièce intermédiaire (5, 105) et à travers l'au moins un tube capillaire (1, 101), et, si présent, également à travers le tuyau (44) jusqu'en dehors de l'au moins une ouverture (2, 102) de l'au moins un tube capillaire (1, 101).

5. Dispositif selon l'une des revendications précédentes, **caractérisé en ce que**
la pièce intermédiaire (5, 105) est en forme de cylindre creux ou en forme de tuyau, dans lequel, de préférence, la pièce intermédiaire (5, 105) présente un rétrécissement, de préférence un rétrécissement conique, sur son côté distal relié à l'au moins un tube capillaire (1, 101), dans lequel, de manière particulièrement préférée, le rétrécissement conique est un cône creux (13, 113), et/ou
la pièce intermédiaire (5, 105) est disposée de manière à être mobile axialement dans le guide (6, 106) lorsque la fixation de l'élément de fixation (8, 108) est relâchée.

6. Dispositif selon l'une des revendications précédentes, **caractérisé en ce que**
le noyau (9) est relié de manière amovible à la pièce intermédiaire (5, 105) au moyen d'un filetage (42) ou d'une fermeture à baïonnette.

7. Dispositif selon l'une des revendications précédentes, **caractérisé en ce que**
l'élément de fixation (8, 108) est un dispositif de serrage comportant des mâchoires de serrage pouvant être vissées ensemble, ou
l'élément de fixation (8, 108) est une vis, dans lequel la vis est vissée ou peut être vissée dans un alésage traversant une paroi du guide (6, 106) et comportant un filetage intérieur, de sorte qu'avec une extrémité avant de la vis, la pièce intermédiaire (5, 105) peut être serrée dans le guide (6, 106).

8. Dispositif selon l'une des revendications précédentes, **caractérisé en ce que**
la pièce intermédiaire (5, 105) est perméable aux liquides lorsque la pièce intermédiaire (5, 105) est fixée dans le guide (6, 106) au moyen de l'élément de fixation (8, 108).

9. Dispositif selon l'une des revendications précédentes, **caractérisé en ce que**
la cavité du guide (6, 106) est au moins aussi longue que la longueur de l'au moins un tube capillaire (1, 101) ou que la longueur du tube capillaire (1, 101) le plus long de l'au moins un tube capillaire (1, 101), et/ou
la cavité dans le guide (6, 106) est effilée au niveau de l'extrémité distale, en particulier est effilée de manière continue ou effilée de manière conique, **et en ce qu'**une percée est disposée au niveau de l'extrémité distale de la zone effilée, en particulier au niveau d'une pointe d'un cône, à travers laquelle percée l'au moins un tube capillaire (1, 101) est guidé ou peut être guidé à travers l'extrémité distale du guide (6, 106), dans lequel, de préférence, le diamètre de la percée est plus petit que le diamètre extérieur de la pièce intermédiaire (5, 105).

10. Dispositif selon l'une des revendications précédentes, **caractérisé en ce que**
le dispositif présente un récipient (28) pour le fluide pharmaceutique, dans lequel, de préférence, un fluide pharmaceutique est contenu dans le récipient (28), de manière particulièrement préférée, le fluide contenant au moins une substance active antibiotique, au moins une substance active antifongique et/ou au moins un agent chimiothérapeutique, et/ou
le noyau (9) fait saillie depuis au moins l'ouverture (2, 102) de l'au moins une ouverture (2, 102) qui est disposée le plus près de l'extrémité distale (3, 103) de l'au moins un tube capillaire (1, 101), dans une mesure telle que, lors d'une flexion de l'au moins un tube capillaire (1, 101), ladite ouverture (2, 102) est fermée sur au moins 50 % de la surface de section transversale de ladite ouverture (2, 102).

11. Dispositif selon l'une des revendications précédentes, **caractérisé en ce que**
l'au moins un tube capillaire (1, 101) est au moins deux tubes capillaires (101), dans lequel les au moins deux tubes capillaires (101) possèdent des longueurs différentes, de préférence tous les tubes capillaires (101) des au moins deux tubes capillaires (101) possèdent des longueurs différentes.

12. Dispositif selon l'une des revendications précédentes, **caractérisé en ce que**
l'au moins un tube capillaire (1, 101) est en titane, en acier inoxydable ou en une matière plastique, en particulier en une matière plastique indéformable, et/ou l'au moins un tube capillaire (1, 101) et le noyau (9) sont déformables, dans lequel l'au moins un tube capillaire (1, 101) est de préférence déformable plastiquement.

13. Dispositif selon l'une des revendications précédentes, **caractérisé en ce que**
un disque poreux et spongieux est disposé au niveau de l'extrémité distale du guide (6, 106), dans lequel, de préférence, le disque poreux et spongieux contient un liquide antiseptique, en particulier une solution antiseptique.

14. Procédé pour la préparation du dispositif selon l'une des revendications précédentes avant une utilisation médicale, dans lequel le procédé n'est pas utilisé pour le traitement du corps humain ou d'un corps animal, le procédé présentant les étapes suivantes se déroulant chronologiquement l'une après l'autre :
A) poussée de l'au moins un tube capillaire (1, 101) hors du guide (6, 106) en insérant la pièce intermédiaire (5, 105) dans le guide (6, 106) ; et
B) fixation de la pièce intermédiaire (5, 105) dans le guide (6, 106) au moyen de l'élément de fixation (8, 108).

15. Procédé selon la revendication 14, **caractérisé en ce que**
dans une étape C) après l'étape B) ou après l'étape A), un formage de l'au moins un tube capillaire (1, 101) par déformation plastique de l'au moins un tube capillaire (1, 101) est effectué.
